(19) Europäisches Patentamt / European Patent Office / Office européen des brevets

(11) **EP 2 823 784 A1**

(12) **EUROPEAN PATENT APPLICATION**
published in accordance with Art. 153(4) EPC

(43) Date of publication:
**14.01.2015 Bulletin 2015/03**

(21) Application number: **13768083.1**

(22) Date of filing: **26.03.2013**

(51) Int Cl.:
**A61B 19/00** (2006.01)    **A61B 1/00** (2006.01)
**A61B 6/03** (2006.01)    **G06T 15/08** (2011.01)

(86) International application number:
**PCT/JP2013/002065**

(87) International publication number:
**WO 2013/145730 (03.10.2013 Gazette 2013/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **29.03.2012   JP 2012077119**

(71) Applicants:
• **Panasonic Healthcare Co., Ltd.**
  **Ehime 791-0395 (JP)**
• **Panasonic Medical Solutions Co. Ltd.**
  **Osaka 571-8504 (JP)**

(72) Inventors:
• **TAKEMURA, Tomoaki**
  **Ehime 791-0395 (JP)**
• **IMANAKA, Ryoichi**
  **Ehime 791-0395 (JP)**
• **IMANISHI, Keiho**
  **Hyogo 663-8179 (JP)**
• **YOSHIDA, Munehito**
  **Wakayama 641-8509 (JP)**
• **KIOKA, Masahiko**
  **Wakayama 641-8509 (JP)**

(74) Representative: **Eisenführ Speiser**
  **Patentanwälte Rechtsanwälte PartGmbB**
  **Johannes-Brahms-Platz 1**
  **20355 Hamburg (DE)**

(54) **SURGERY ASSISTANCE DEVICE AND SURGERY ASSISTANCE PROGRAM**

(57)    A personal computer (1) of a surgery assistance system (100) performs navigation during surgery while combining and displaying an image of the distal end (surgical instrument image (33a)) of a surgical instrument (33) and a distance from the surgical instrument distal end to the resection site, in a three-dimensional image produced by a volume rendering computer (13).

FIG. 3

EP 2 823 784 A1

**Description**

TECHNICAL FIELD

The present invention relates to a surgery assistance device and a surgery assistance program with which navigation during surgery is performed.

BACKGROUND ART

In a medical facility, surgery assistance devices that allow surgery to be simulated are employed in order to perform better surgery.

A conventional surgery assistance device comprised, for example, a tomographic image information acquisition section for acquiring tomographic image information, such as an image acquired by PET (positron emission tomography), a nuclear magnetic resonance image (MRI), or an X-ray CT image, a memory connected to the tomographic image information acquisition section, a volume rendering computer connected to the memory, a display for displaying the computation results of the volume rendering computer, and an input section for giving resecting instructions with respect to a displayed object that is being displayed on the display.

For example, Patent Literature 1 discloses an endoscopic surgery assistance device with which the coordinates of a three-dimensional image of the endoscope actually being used and the coordinates of three-dimensional volume image data produced using a tomographic image are integrated, and these are displayed superposed over endoscopic video, which allows an image of the surgical site region to be displayed superposed at this location over an endoscopic image in real time, according to changes in the endoscope or surgical instrument.

CITATION LIST

PATENT LITERATURE

Patent Literature 1: Japanese Patent No. 4,152,402 (registered July 11, 2008)

SUMMARY

TECHNICAL PROBLEM

However, the following problem was encountered with the conventional surgery assistance device discussed above.

Specifically, with the surgery assistance device disclosed in the above publication, since an image of the surgical site region displayed superposed at that location over endoscopic image in real time, the distance between the surgical instrument distal end and a specific region can be calculated. What is disclosed here, however, does not involve navigation during surgery, and is just a warning and a display of the distance to the site of a blood vessel, organ, or the like with which the surgical instrument must not come into contact.

It is an object of the present invention to provide a surgery assistance device and a surgery assistance program with which proper navigation can be performed during surgery while the user views the resection site, which is resected using a surgical instrument.

SOLUTION TO PROBLEM

The surgery assistance device pertaining to the first invention is a surgery assistance device for performing navigation while displaying a three-dimensional simulation image produced from tomographic image information during surgery in which a resection-use surgical instrument is used while the user views an endoscopic image, the device comprising a tomographic image information acquisition section, a memory, a volume rendering computer, an endoscope/surgical instrument position sensor, a registration computer, a simulator, a distance calculator, and a navigator. The tomographic image information acquisition section acquires tomographic image information about a patient. The memory is connected to the tomographic image information acquisition section and stores voxel information for the tomographic image information. The volume rendering computer is connected to the memory and samples voxel information in a direction perpendicular to the sight line on the basis of the voxel information. The endoscope/surgical instrument position sensor sequentially senses the three-dimensional positions of the endoscope and the surgical instrument. The registration computer integrates the coordinates of a three-dimensional image produced by the volume rendering computer and the coordinates of the endoscope and the surgical instrument sensed by the endoscope/surgical instrument position sensor. The simulator stores the resection portion scheduled for surgery and virtually resected on the three-dimensional image produced by the volume rendering computer, in the memory after associating it with the voxel information. The distance calculator calculates a distance between the working end of the surgical instrument on the three-dimensional image and the voxel information indicating the resection portion and stored in the memory. The navigator displays the working end of the surgical instrument on the three-dimensional image by using the coordinates of the surgical instrument during surgery, and displays the distance between the working end and the voxel information indicating the resection portion stored in the memory, along with the endoscopic image displayed during surgery.

Here, for example, after a resection simulation is conducted in a state in which the area around a specific bone, blood vessel, organ, or the like is displayed using a three-dimensional image produced from a plurality of X-ray CT images, when surgery is performed using an endoscope, three-dimensional positions of the endoscope or surgical instrument actually used in the surgery are sequentially sensed, and the coordinates of a three-dimensional image formed from a plurality of X-ray CT images and the coordinates of the actual three-dimensional position of the endoscope and the surgical instrument are integrated. Then, the distance to the distal end (the working end) of the actual surgical instrument with respect to the site to be resected in the resection simulation performed using a three-dimensional image is calculated, and this distance is displayed along with the three-dimensional image to advise the surgeon, so that surgical navigation is performed seamlessly from the resection simulation.

Here, the above-mentioned tomographic image includes, for example, two-dimensional images acquired using X-ray CT, MRI, PET, or another such medical device. The above-mentioned surgical instrument includes resection instruments for resecting organs, bones, and so forth. The above-mentioned "working end" means the tooth portion, etc., of the surgical instrument that cuts out the bone, organ, or the like.

Consequently, in surgery for resecting a specific organ by using an endoscope, for example, the surgeon can accurately ascertain how far the distal end of the surgical instrument is from the site that is to be resected, while moving the resection instrument or other surgical instrument toward the resection site. This allows the surgeon to navigate properly while inserting the surgical instrument, without feeling any uncertainty due to not knowing how far apart the surgical instrument distal end and the resection site are.

The surgery assistance device pertaining to the second invention is the surgery assistance device pertaining to the first invention, wherein the simulator senses the depth of the surgical site during pre-surgery resection simulation and computes the degree of change in depth or discontinuity, and stops the resection or does not update the resection data if the degree of change exceeds a specific threshold.

Here, the simulator sets a threshold for virtual resection, and provides a restriction when resection simulation is performed.

Consequently, if the change in depth, etc., exceeds the threshold, the site will not be displayed in a post-resection state on the simulation image. Also, this avoids a situation in which the threshold value becomes too small, or the resection is halted too much, when the resection simulation is performed while the threshold is updated.

The surgery assistance device pertaining to the third invention is the surgery assistance device pertaining to the first or second invention, wherein the navigator models, by multi-point model, the working end of the surgical instrument on the three-dimensional image.

Here, the multi-point model is a model for sampling a plurality of points on the outer edge of the site where collision is expected to occur.

Consequently, when a sensor for sensing the position, angle, etc., is provided to the surgical instrument at a specific position of the actual surgical instrument, for example, the surgical instrument will be represented by multiple points in a virtual space, using the position of this sensor as a reference, and the distance to the resection portion can be calculated from these multiple points and displayed.

The surgery assistance device pertaining to the fourth invention is the surgery assistance device pertaining to any of the first to third inventions, wherein the navigator uses a vector that has a component of the direction of voxel information indicating the resected portion by the surgical instrument during surgery as the vector of the distance.

Consequently, sampling can be performed in the direction in which the surgical instrument moves closer to the resection site, while the positional relation between the resection site and the surgical instrument distal end with respect to the surgeon can be more effectively displayed, such as changing the display mode according to the speed, acceleration, and direction at which the multiple points approach.

The surgery assistance device pertaining to the fifth invention is the surgery assistance device pertaining to any of the first to fourth inventions, wherein the navigator changes the display color of the voxels for each equidistance from the resection portion.

Here, the range of equidistance, centered on the resection portion, is displayed as spheres of different colors on the navigation screen during surgery.

Consequently, in navigation during surgery, the surgeon can easily see the distance from the portion where resection is performed to the surgical instrument distal end, which facilitates navigation.

The surgery assistance device pertaining to the sixth invention is the surgery assistance device pertaining to any of the first to fifth inventions, wherein, after integrating the coordinates of a three-dimensional image and the coordinates of the endoscope and the surgical instrument, the registration computer checks the accuracy of this coordinate integration, and corrects deviation in the coordinate integration if this accuracy exceeds a specific range.

Here, the accuracy of registration in which the coordinates of the three-dimensional image produced on the basis of a plurality of X-ray CT images, etc., and the actual coordinates of the endoscope and surgical instrument is checked, and registration is performed again if a specific level of accuracy is not met.

This allows the position of the endoscope or surgical instrument displayed in the three-dimensional image to be displayed more accurately in the three-dimensional image.

The surgery assistance device pertaining to the seventh invention is the surgery assistance device pertaining to any of the first to sixth inventions, wherein the navigator sets and displays a first display area acquired by the endoscope and produced by the volume rendering computer, and a second display area in which the display is restricted by the surgical instrument during actual surgery.

Here, in the three-dimensional image displayed on the monitor screen, etc., during surgery, the display shows the portion of the field of view that is restricted by the surgical instrument into which the endoscope is inserted.

Therefore, the display is in a masked state, for example, so that the portion restricted by the retractor or other such tubular surgical instrument cannot be seen, and this allows a three-dimensional image to be displayed in a state that approximates the actual endoscopic image.

The surgery assistance device pertaining to the eighth invention is the surgery assistance device pertaining to any of the first to seventh inventions, further comprising a display component that displays the three-dimensional image, an image of the distal end of the surgical instrument, and the distance.

The surgery assistance device here comprises a monitor or other such display component.

Therefore, surgery can be assisted while a three-dimensional image that approximates the actual video from an endoscope is displayed on the display component during surgery in which an endoscope is used.

The surgery assistance program pertaining to the ninth invention is a surgery assistance program that performs navigation while displaying a three-dimensional simulation image produced from tomographic image information, during surgery in which a resection-use surgical instrument is used while an endoscopic image, wherein the surgery assistance program is used by a computer to execute a surgery assistance method comprising the steps of acquiring tomographic image information about a patient, storing voxel information for the tomographic image information, sampling voxel information in a direction perpendicular to the sight line on the basis of the voxel information, sequentially sensing the three-dimensional positions of the endoscope and surgical instrument, integrating the coordinates of the three-dimensional image and the coordinates of the endoscope and the surgical instrument, calculating the distance between the working end of the surgical instrument and the resection site included in the video acquired by the endoscope, and displaying the working end of the surgical instrument on the three-dimensional image by using the coordinates of the surgical instrument during surgery, and combining and displaying an image indicating the distal end of the surgical instrument, and the distance between the resection site and the distal end of the surgical instrument, while navigation is performed during surgery.

Here, for example, after a resection simulation is conducted in a state in which the area around a specific bone, blood vessel, organ, or the like is displayed using a three-dimensional image produced from a plurality of X-ray CT images, when surgery is performed using an endoscope, three-dimensional positions of the endoscope or surgical instrument actually used in the surgery are sequentially sensed, and the coordinates of a three-dimensional image formed from a plurality of X-ray CT images and the coordinates of the actual three-dimensional position of the endoscope and the surgical instrument are integrated. Then, the distance to the distal end of the actual surgical instrument with respect to the site to be resected in the resection simulation performed using a three-dimensional image is calculated, and this distance is displayed along with the three-dimensional image to advise the surgeon, so that surgical navigation is performed seamlessly from the resection simulation.

Here, the above-mentioned tomographic image includes, for example, two-dimensional images acquired using X-ray CT, MRI, PET, or another such medical device. The above-mentioned surgical instrument includes resection instruments for resecting organs, bones, and so forth.

Consequently, in surgery for resecting a specific organ by using an endoscope, for example, the surgeon can accurately ascertain how far the distal end of the surgical instrument is from the site that is to be resected, while moving the resection instrument or other surgical instrument toward the resection site. This allows the surgeon to navigate properly while inserting the surgical instrument, without feeling any uncertainty due to not knowing how far apart the surgical instrument distal end and the resection site are.

The surgery assistance device pertaining to the tenth invention is a surgery assistance device for performing navigation while displaying a three-dimensional simulation image produced from tomographic image information, during surgery in which a resection-use surgical instrument is used while the user views an endoscopic image, the device comprising a simulator and a navigator. The simulator stores the resection portion scheduled for surgery and virtually resected on the three-dimensional image produced by sampling voxel information for the tomographic image information of the patient in a direction perpendicular to the sight line, after associating it with the voxel information. The navigator calculates a distance between the working end of the surgical instrument on the three-dimensional image and the voxel information indicating the resection portion stored in the memory, displays the working end of the surgical instrument on the three-dimensional image by using the coordinates of the surgical instrument during surgery, and displays the distance between the working end and the voxel information indicating the resection portion, along with the endoscopic image displayed during surgery.

BRIEF DESCRIPTION OF DRAWINGS

FIG. 1 shows the configuration of a surgery assistance system that includes a personal computer (surgery assistance device) pertaining to an embodiment of the present invention;

FIG. 2 is an oblique view of the personal computer included in the surgery assistance system in FIG. 1;

FIG. 3 is a control block diagram of the personal computer in FIG. 2;

FIG. 4 is a block diagram of the configuration of an endoscope parameter storage section in a memory included in the control blocks in FIG. 3;

FIG. 5 is a block diagram of the configuration of an endoscope parameter storage section in the memory included in the control blocks in FIG. 3;

FIGS. 6A and 6B are a side view and a plan view of an oblique endoscope included in the surgery assistance system in FIG. 1 and a three-dimensional sensor attached to this endoscope;

FIG. 7A is an operational flowchart of the personal computer in FIG. 2, FIG. 7B is an operational flowchart of the flow in S6 of FIG. 7A, and FIG. 7C is an operational flowchart of the flow in S8 in FIG. 7A;

FIG. 8 shows a navigation screen displayed on the display of the surgery assistance system in FIG. 1;

FIG. 9 shows a navigation screen displayed on the display of the surgery assistance system in FIG. 1;

FIG. 10 shows a navigation screen displayed on the display of the surgery assistance system in FIG. 1;

FIG. 11 shows a navigation screen displayed on the display of the surgery assistance system in FIG. 1;

FIG. 12 shows a navigation screen displayed on the display of the surgery assistance system in FIG. 1;

FIGS. 13A and 13B illustrate mapping from two-dimensional input with a mouse to three-dimensional operation with an endoscope when a tubular surgical instrument (retractor) is used;

FIG. 14 illustrates mapping from two-dimensional input with a mouse to three-dimensional operation with an endoscope;

FIG. 15 illustrates the display of a volume rendering image that shows the desired oblique angle with an oblique endoscope;

FIGS. 16A to 16C show displays when the distal end position of an oblique endoscope and the sight line vector are shown on in a three-panel view;

FIG. 17 shows an oblique endoscopic image that is displayed by the personal computer in FIG. 2;

FIG. 18A shows an oblique endoscopic image pertaining to this embodiment, and FIG. 18B shows an endoscopic image when using a direct-view endoscope instead of an oblique endoscope;

FIG. 19 shows a monitor screen that shows the restricted display area of an oblique endoscope;

FIGS. 20A to 20C show an endoscopic image centered on a resection site C, an endoscopic view cropped from a three-dimensional image corresponding to a portion of this site, and a monitor screen displaying an image in which the endoscopic image and the endoscopic view are superposed;

FIGS. 21A to 21C show an endoscopic image, a three-dimensional image (VR image) corresponding to that portion, and a monitor screen displaying an image in which the endoscopic image and the VR image are superposed;

FIG. 22 shows a monitor screen displaying a registration interface screen for setting feature points;

FIG. 23 illustrates coordinate conversion in registration;

FIGS. 24A and 24B show a correction value setting interface in registration, and a display example of the coordinate axis and feature points on a volume rendering image;

FIG. 25A is a side view of a surgical instrument included in the surgery assistance system in FIG. 1, and a three-dimensional sensor attached thereto, and FIG. 25B is a side view in which the distal end of a surgical instrument is modeled by multi-point modeling in a virtual space in which the sensor in FIG. 25A is used as a reference;

FIG. 26 illustrates the step of calculating and displaying the distance from the distal end of the surgical instrument in FIG. 25B to the resection site;

FIG. 27 shows a display example in which a region of equidistance from the resection site in virtual space is displayed;

FIG. 28 illustrates a case in which resection control encompassing the concept of threshold summing valid points is applied to a method for updating a threshold in which resection is restricted in resection simulation;

FIG. 29 illustrates a case in which resection control not encompassing the concept of threshold summing valid points is applied to a method for updating a threshold in which resection is restricted in resection simulation;
FIGS. 30A and 30B are a side view and a plan view showing an endoscope and sensor used in the surgery assistance system pertaining to another embodiment of the present invention; and
FIGS. 31A and 31B are a side view and a plan view showing an endoscope and sensor used in the surgery assistance system pertaining to yet another embodiment of the present invention.

DESCRIPTION OF EMBODIMENTS

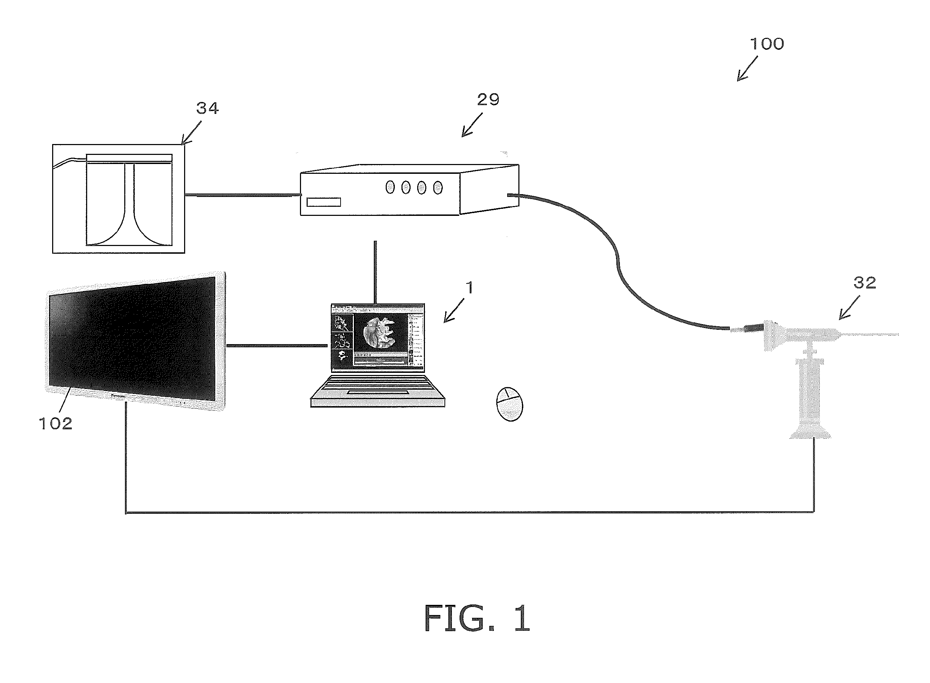

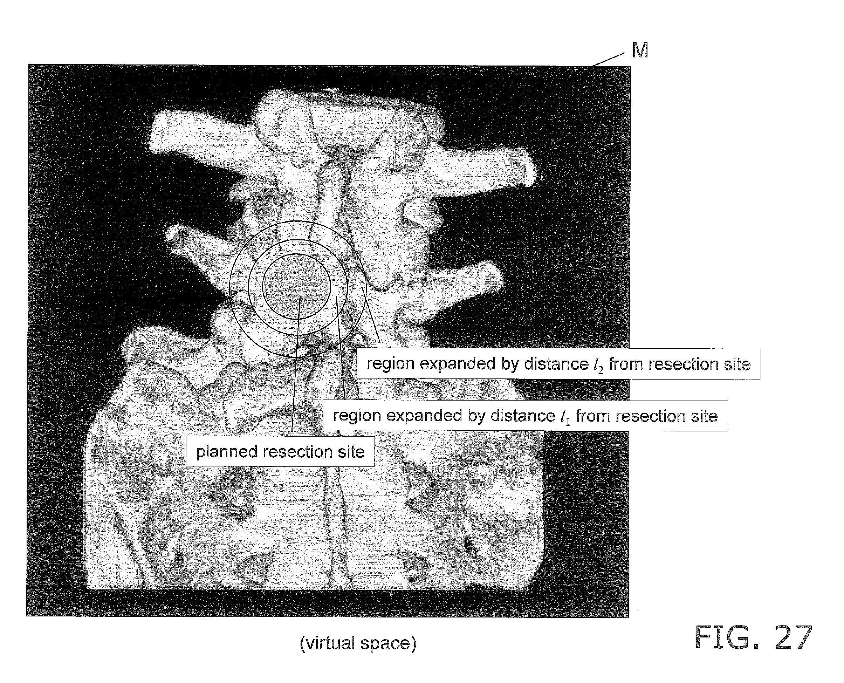

The personal computer (surgery assistance device) pertaining to an embodiment of the present invention will now be described through reference to FIGS. 1 to 29.

In this embodiment, a case is described in which navigation is performed in surgery for lumbar spinal stenosis using an endoscope and a resection tool or other such surgical instrument, but the present invention is not limited to this.

As shown in FIG. 1, the personal computer 1 pertaining to this embodiment constitutes a surgery assistance system 100 along with a display (display component) 2, a position and angle sensing device 29, an oblique endoscope (endoscope) 32, and a positioning transmitter (magnetic field generator) 34.

The personal computer 1 functions as a surgery assistance device by reading a surgery assistance program that causes a computer to execute the surgery assistance method of this embodiment. The configuration of the personal computer 1 will be discussed in detail below.

The display (display component) 2 displays a three-dimensional image for performing resection simulation or navigation during surgery (discussed below), and also displays a setting screen, etc., for surgical navigation or resection simulation.

Since the display component for displaying navigation during surgery needs to display a navigation screen that is easy for the surgeon to understand during surgery, a large liquid crystal display 102 that is included in the surgery assistance system 100 in FIG. 1 is also used in addition to the display 2 of the personal computer 1.

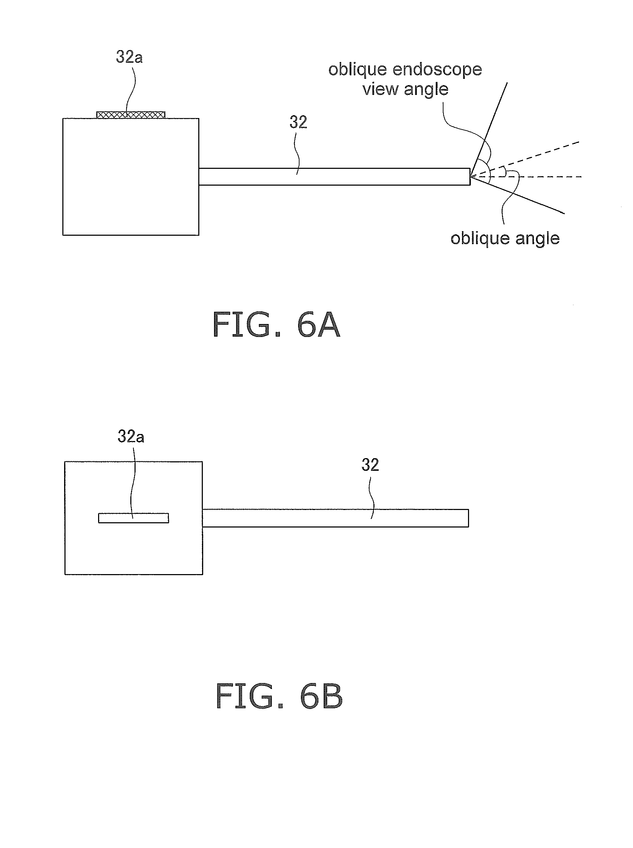

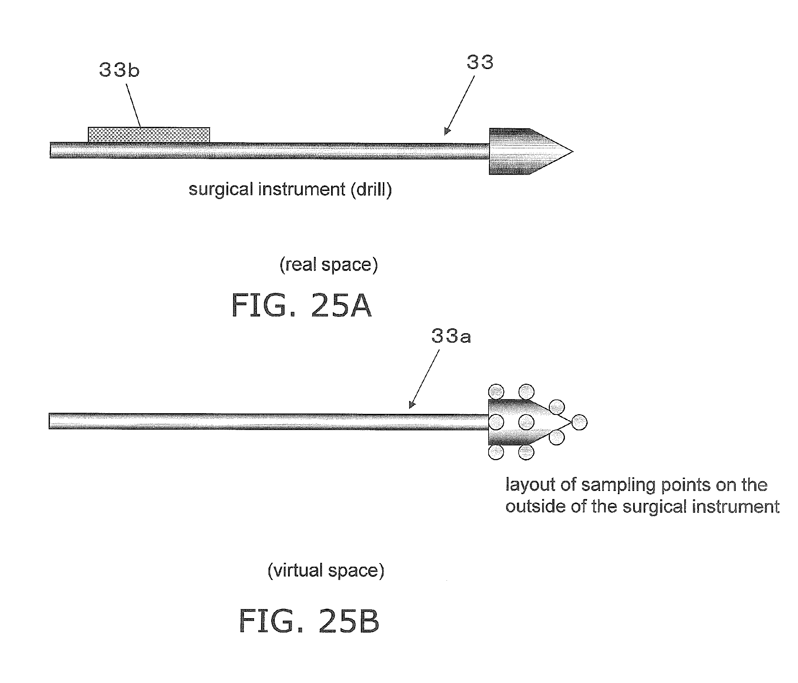

The position and angle sensing device 29 is connected to the personal computer 1, the positioning transmitter 34, and the oblique endoscope 32, and the position and attitude of the oblique endoscope 32 or the surgical instrument 33 during actual surgery are sensed on the basis of the sensing result of a three-dimensional sensor 32a (see FIG. 6A, etc.) or a three-dimensional sensor 33b (see FIG. 25A) attached to the oblique endoscope 32, the surgical instrument 33, etc.

The oblique endoscope (endoscope) 32 is inserted from the body surface near the portion undergoing surgery, into a tubular retractor 31 (discussed below), and acquires video of the surgical site. The three-dimensional sensor 32a is attached to the oblique endoscope 32.

The positioning transmitter (magnetic field generator) 34 is disposed near the surgical table on which the patient is lying, and generates a magnetic field. Consequently, the position and attitude of the oblique endoscope 32 and the surgical instrument 33 can be sensed by sensing the magnetic field generated by the positioning transmitter 34 at the three-dimensional sensor 32a or the three-dimensional sensor 33b attached to the oblique endoscope 32 and the surgical instrument 33.

Personal Computer 1

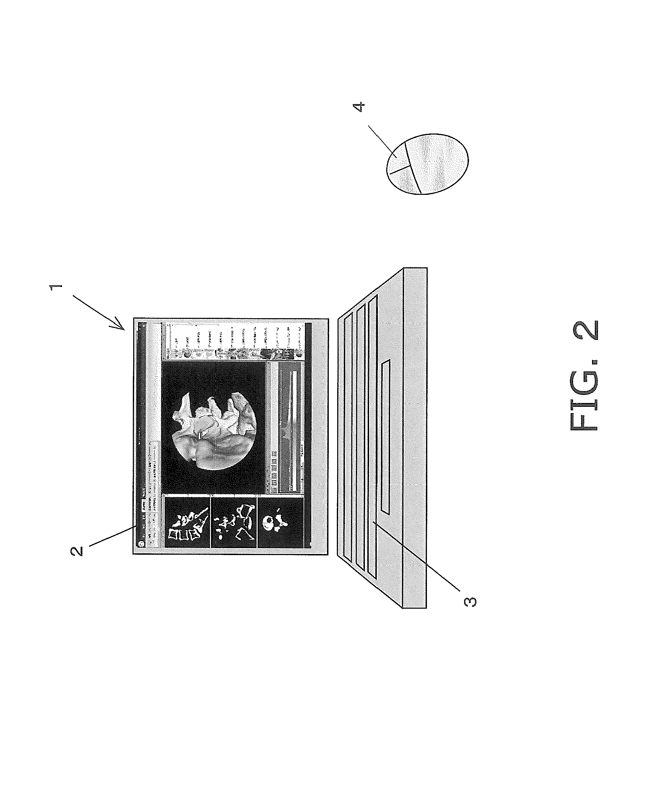

As shown in FIG. 2, the personal computer 1 comprises the display (display component) 2 and various input components (a keyboard 3, a mouse 4, and a tablet 5 (see FIG. 2)).

The display 2 displays three-dimensional images of bones, organs, or the like formed from a plurality of tomographic images such as X-ray CT images (an endoscopic image is displayed in the example in FIG. 2), and also displays the results of resection simulation and the content of surgical navigation.

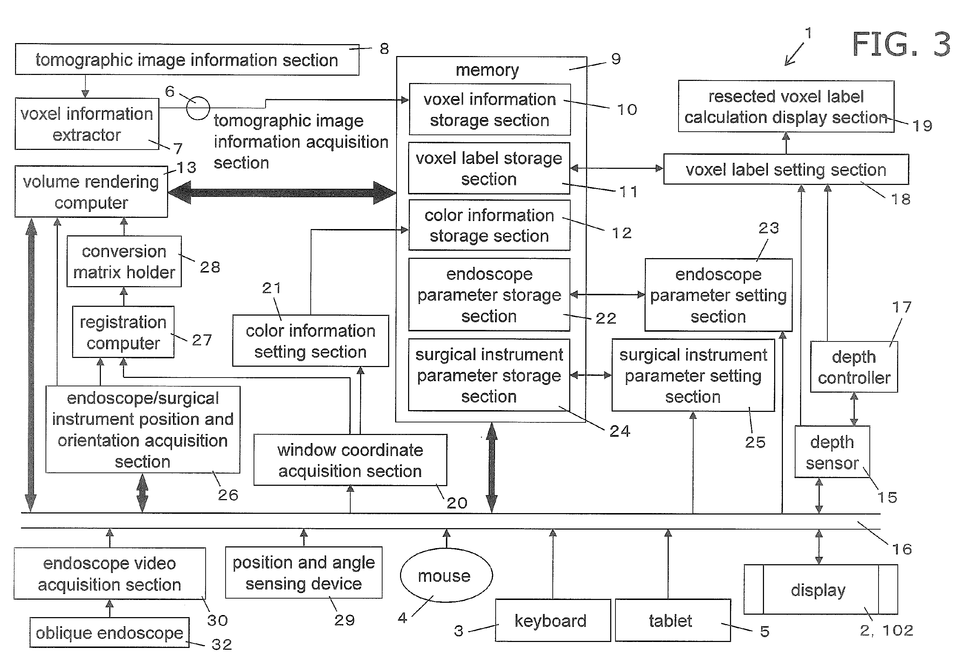

As shown in FIG. 3, control blocks such as the tomographic image information acquisition section 6 are formed in the interior of the personal computer 1.

The tomographic image information acquisition section 6 is connected via the voxel information extractor 7 to the tomographic image information section 8. That is, the tomographic image information section 8 is supplied with tomographic image information from a device that captures tomographic images, such as CT, MRI, or PET, and this tomographic image information is extracted as voxel information by the voxel information extractor 7.

The memory 9 is provided inside the personal computer 1, and has the voxel information storage section 10, the voxel label storage section 11, the color information storage section 12, the endoscope parameter storage section 22, and the surgical instrument parameter storage section 24. The memory 9 is connected to the volume rendering computer 13 (distance calculator, display controller).

The voxel information storage section 10 stores voxel information received from the voxel information extractor 7 via the tomographic image information acquisition section 6.

The voxel label storage section 11 has a first voxel label storage section, a second voxel label storage section, and a third voxel label storage section. These first to third voxel label storage sections are provided corresponding to a predetermined range of CT values (discussed below), that is, to the organ to be displayed. For instance, the first voxel label storage section corresponds to a range of CT values displaying a liver, the second voxel label storage section corresponds to a range of CT values displaying a blood vessel, and the third voxel label storage section corresponds to a range of CT values displaying a bone.

The color information storage section 12 has a plurality of storage sections in its interior. These storage sections are each provided corresponding to a predetermined range of CT values, that is, to the bone, blood vessel, nerve, organ, or the like to be displayed. For instance, there may be a storage section corresponding to a range of CT values displaying a liver, a storage section corresponding to a range of CT values displaying a blood vessel, and a storage section corresponding to a range of CT values displaying a bone. Here, the various storage sections are set to different color information for each of the bone, blood vessel, nerve, or organ to be displayed. For example, white color information may be stored for the range of CT values corresponding to a bone, and red color information may be stored for the range of CT values corresponding to a blood vessel.

The CT values set for the bone, blood vessel, nerve, or organ to be displayed is the result of digitizing the extent of X-ray absorption in the body, and is expressed as a relative value (in units of HU), with water at zero. For instance, the range of CT values in which a bone is displayed is 500 to 1000 HU, the range of CT values in which blood is displayed is 30 to 50 HU, the range of CT values in which a liver is displayed is 60 to 70 HU, and the range of CT values in which a kidney is displayed is 30 to 40 HU.

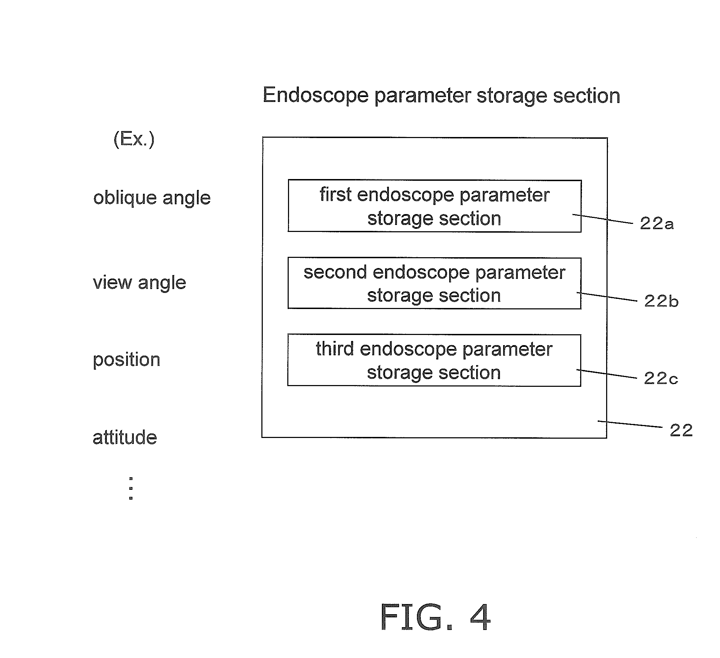

As shown in FIG. 4, the endoscope parameter storage section 22 has a first endoscope parameter storage section 22a, a second endoscope parameter storage section 22b, and a third endoscope parameter storage section 22c. The first to third endoscope parameter storage sections 22a to 22c store endoscope oblique angles, viewing angles, positions, attitudes, and other such information. The endoscope parameter storage section 22 is connected to an endoscope parameter setting section 23, as shown in FIG. 3.

The endoscope parameter setting section 23 sets the endoscope parameters inputted via the keyboard 3 or the mouse 4, and sends them to the endoscope parameter storage section 22.

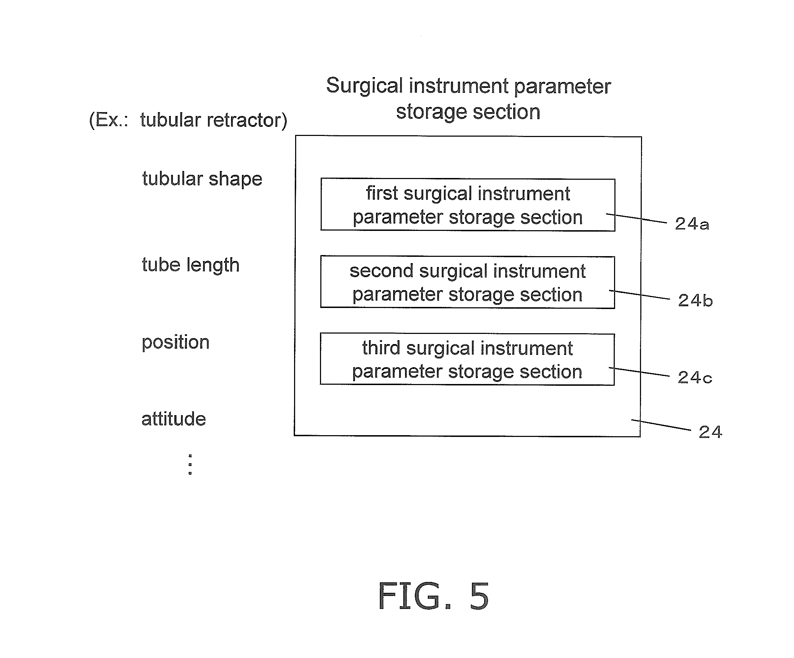

As shown in FIG. 5, the surgical instrument parameter storage section 24 has a first surgical instrument parameter storage section 24a, a second surgical instrument parameter storage section 24b, and a third surgical instrument parameter storage section 24c. The first to third surgical instrument parameter storage sections 24a to 24c each store information such as the length, distal end shape, position, and attitude of the drill (if the surgical instrument is a drill), for example. As shown in FIG. 2, the surgical instrument parameter storage section 24 is connected to a surgical instrument parameter setting section 25.

The surgical instrument parameter setting section 25 sets surgical instrument parameters for the retractor 31, drill, etc., that are inputted via the keyboard 3 or the mouse 4, and sends them to the surgical instrument parameter storage section 24.

An endoscope/surgical instrument position and attitude acquisition section (endoscope/surgical instrument position sensor) 26 receives via a bus 16 the sensing result from the position and angle sensing device 29, which senses the position and angle of the endoscope or surgical instrument, and sends this result to the volume rendering computer 13 and a registration computer 27.

The volume rendering computer 13 acquires a plurality of sets of slice information at a specific spacing in the Z direction and perpendicular to the sight line, on the basis of the voxel information stored in the voxel information storage section 10, the voxel labels stored in the voxel label storage section 11, and the color information stored in the color information storage section 12. The volume rendering computer 13 then displays this computation result as a three-dimensional image on the display 2.

The volume rendering computer 13 also gives a real-time display that combines the movements of the actual endoscope or surgical instrument into a three-dimensional image on the basis of endoscope information stored in the endoscope parameter storage section 22, surgical instrument information stored in the surgical instrument parameter storage section 24, and the sensing result from the endoscope/surgical instrument position and attitude acquisition section 26.

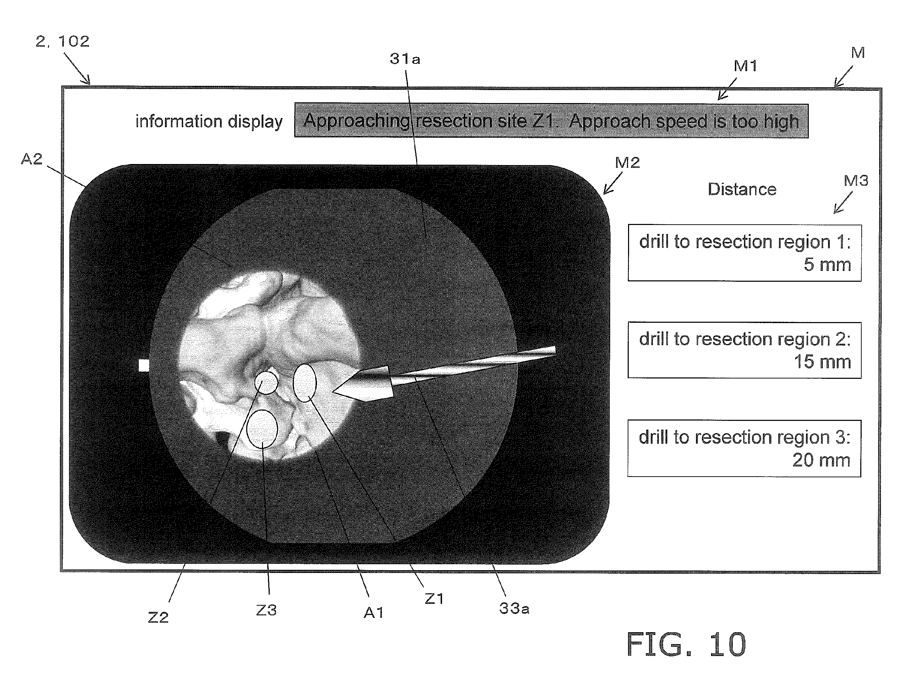

The volume rendering computer 13 also displays a virtual endoscopic image on the display 2 in a masked state that reflects image information in which the field of view is restricted by the retractor 31, with respect to the image information obtained by the endoscope, on the basis of the above-mentioned endoscopic information and surgical instrument information. More specifically, the volume rendering computer 13 sets an endoscopic image display area (first display area) A1 (see FIG. 10, etc.) acquired by the endoscope, and a restricted display area (second display area) A2 (see FIG. 10, etc.), on the basis of information related to the endoscope stored in the endoscope parameter storage section 22 (oblique angle, view angle, position, etc.) and information related to the surgical instrument stored in the surgical instrument parameter storage section 24 (diameter, length, etc.).

The endoscopic image display area A1 here is a display area that is displayed on the monitor screen of the display 2 during actual endoscopic surgery. The restricted display area A2 is a display area in which the display acquired by the endoscope is restricted by the inner wall portion, etc., of the surgical instrument, such as a tubular retractor 31, and refers to a region whose display is masked in endoscopic surgery simulation (see FIG. 10, etc.).

The volume rendering computer 13 is also connected to a depth sensor 15 via the bus 16.

The depth sensor 15 measures the ray casting scanning distance, and is connected to a depth controller 17 and a voxel label setting section 18.

The voxel label setting section 18 is connected to the voxel label storage section 11 and to a resected voxel label calculation display section 19.

In addition to the above-mentioned volume rendering computer 13 and depth sensor 15, the bus 16 is also connected to the endoscope/surgical instrument position and attitude acquisition section 26 and a window coordinate acquisition section 20, such as the color information storage section 12 in the memory 9, and displays three-dimensional images and so forth on the display 2 on the basis of what is inputted from the keyboard 3, the mouse 4, the tablet 5, the position and angle sensing device 29, an endoscope video acquisition section 30, and so on.

The window coordinate acquisition section 20 is connected to a color information setting section 21 and the registration computer 27.

The color information setting section 21 is connected to the color information storage section 12 in the memory 9.

As discussed above, the endoscope/surgical instrument position and attitude acquisition section 26 acquires information related to the positions of the oblique endoscope 32 and the surgical instrument 33 by detecting the magnetic field generated by the positioning transmitter 34 at the three-dimensional sensor 32a and the three-dimensional sensor 33b attached to the oblique endoscope 32 and the surgical instrument 33.

As shown in FIGS. 6A and 6B, the three-dimensional sensor 32a that is used to sense the position and attitude of the oblique endoscope 32 in three dimensions is provided at a position where it will not hinder the operation of the handle of the oblique endoscope 32. Also, as shown in FIG. 25A, the three-dimensional sensor 33b that is used to sense the position and attitude of the surgical instrument 33 in three dimensions is provided at a position where it will not hinder the operation of the handle of the surgical instrument 33.

The registration computer 27 performs computation to match the three-dimensional image produced by the volume rendering computer 13 with the rotational angle and the three-dimensional image of the oblique endoscope 32 and the surgical instrument 33 and the reference position of the patient during actual surgery. The registration processing (coordinate conversion processing) performed by the registration computer 27 will be discussed in detail below.

A conversion matrix holder 28 is connected to the registration computer 27 and the volume rendering computer 13, and holds a plurality of conversion matrixes used in registration processing (coordinate conversion processing).

As discussed above, the position and angle sensing device 29 is connected to the personal computer 1, the positioning transmitter 34, and the oblique endoscope 32, and senses the position and attitude of the oblique endoscope 32 and the surgical instrument 33 during actual surgery on the basis of the sensing result at the three-dimensional sensor 32a (see FIG. 6A, etc.) and the three-dimensional sensor 33b attached to the oblique endoscope 32, the surgical instrument 33, etc.

The endoscope video acquisition section 30 acquires video acquired by the oblique endoscope 32. The endoscope video acquired by the endoscope video acquisition section 30 is displayed on the display 2 and a display 102 via the bus 16.

As discussed above, the retractor 31 is a tubular member into which the oblique endoscope 32 or the surgical instrument 33 (such as a drill) is inserted, and in actual surgery it is inserted into and fixed in the body of the patient from the body surface near the surgical site.

The oblique endoscope (endoscope) 32 is inserted along the inner peripheral face of the above-mentioned tubular retractor 31, and acquires video of the surgical site. The three-dimensional sensor 32a is attached to the oblique endoscope 32 in order to sense the three-dimensional position or attitude of the oblique endoscope 32 in real time during surgery.

As shown in FIGS. 6A and 6B, a single three-dimensional sensor 32a is provided to the side face on the rear end side of the oblique endoscope 32. Thus, the distal end position of the oblique endoscope 32 is calculated on the basis of the length and shape of the oblique endoscope 32, which are stored in the endoscope parameter storage section 22. In this embodiment, a single six-axis sensor is used as the three-dimensional sensor 32a. Therefore, six parameters, namely, (x, y, z), y (yaw), p (pitch), and r (roll), can be measured with just the one three-dimensional sensor 32a.

The surgical instrument 33 in this embodiment is a drill that resects the surgical site. Similar to the oblique endoscope 32, the three-dimensional sensor 33b is attached to the surgical instrument (drill) 33 near the rear end. Consequently, the position of the distal end (working end) of the surgical instrument (drill) 33 doing the resection can also be calculated on the basis of the length and shape of the drill stored in the surgical instrument parameter storage section 24.

More specifically, as shown in FIG. 25A, the three-dimensional sensor 33b is attached at a position in real space where it will not hinder the handle of the surgical instrument 33 used in actual surgery, and the distal end position of a surgical instrument image 33a in virtual space is modeled by multi-point modeling as shown in FIG. 25B.

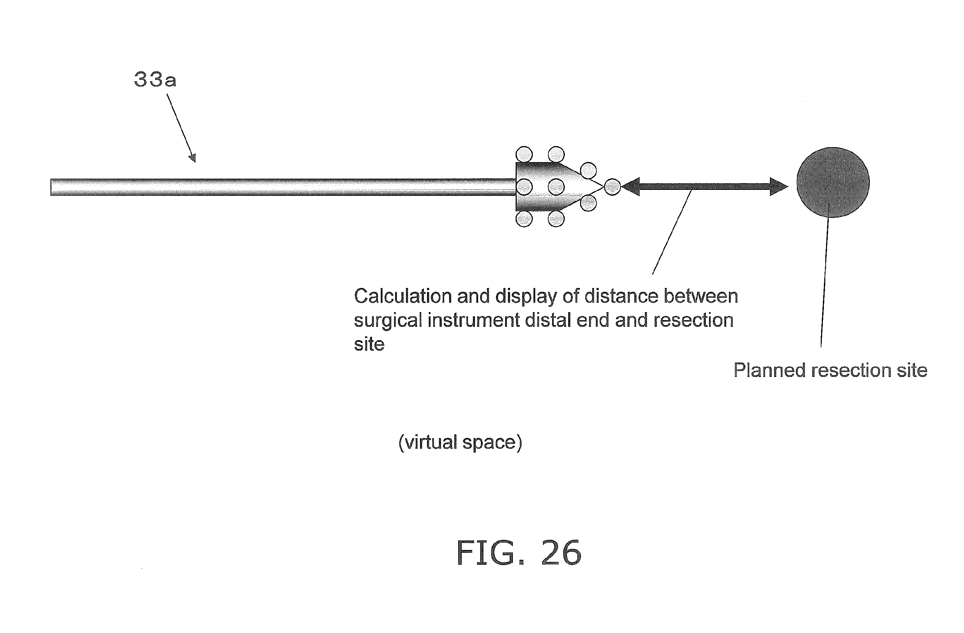

As shown in FIG. 26, the distance in virtual space from the multiple points of the distal end of the surgical instrument 33 to the resection site planned for the surgery is calculated and displayed on the basis of the result of sensing the position, attitude, etc., of the surgical instrument 33 in real time and in conjunction with the operation of the actual surgical instrument 33.

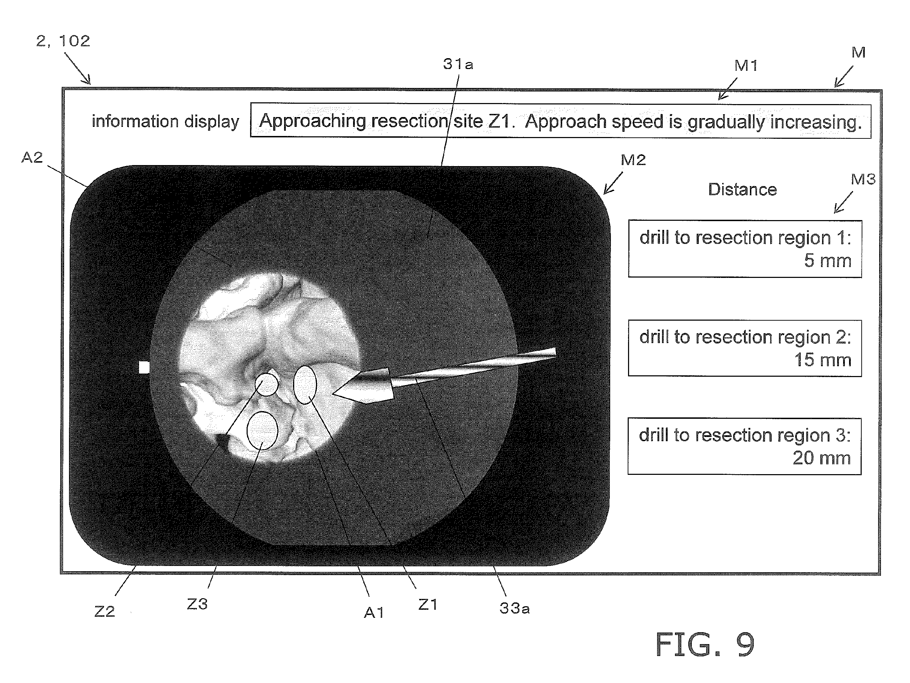

The distance from the multiple points of the distal end of the surgical instrument 33 to the resection site planned for the surgery is sampled in the approaching direction, and the display mode is changed according to the speed, acceleration, and direction at which the multiple points approach (see FIGS. 9 and 10).

Consequently, the surgeon can ascertain the position of the surgical instrument distal end with respect to the resection site more accurately while looking at the image indicating the virtual space used for navigation.

Control Flow Related to this Surgery Assistance Method

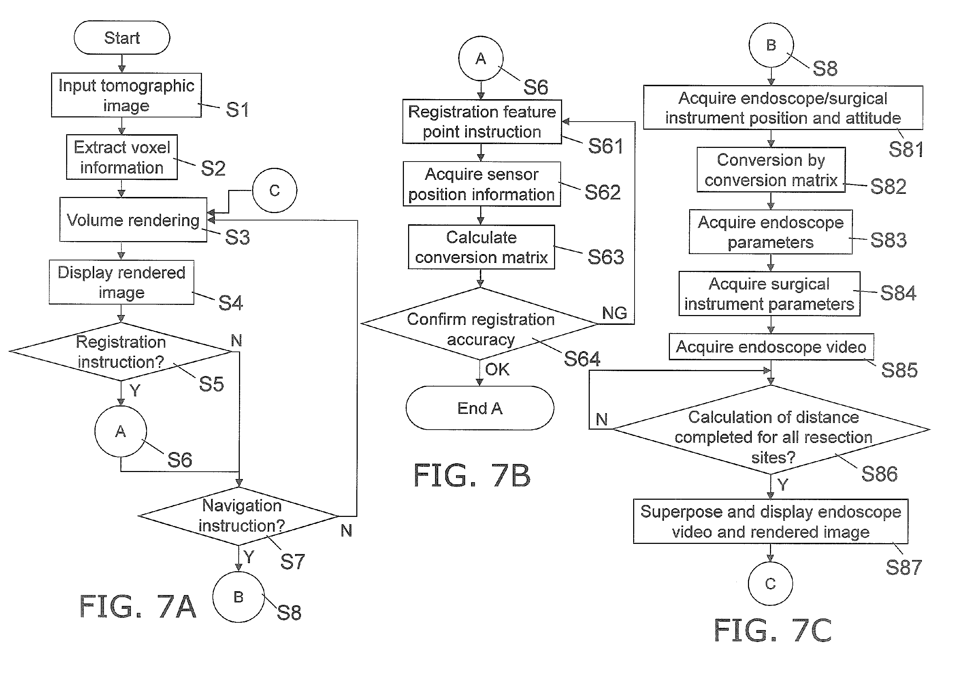

The control flow in the surgery assistance method pertaining to the personal computer 1 in this embodiment will now be described through reference to FIGS. 7A to 7C.

As shown in FIG. 7A, with the personal computer 1 in this embodiment, first, in S1, tomographic image information is inputted from the tomographic image information section 8, and this is supplied to the voxel information extractor 7.

Then, in S2, the voxel information extractor 7 extracts voxel information from the tomographic image information. The extracted voxel information is sent through the tomographic image information acquisition section 6 and stored in the voxel information storage section 10 of the memory 9. Voxel information stored in the voxel information storage section 10 is information about the points made up of $I(x,y,z,\alpha)$, for example. I here is brightness information about these points, while x, y, and z are coordinate points, and $\alpha$ is transparency information.

Then, in S3, the volume rendering computer 13 calculates a plurality of sets of slice information at a specific spacing in the Z direction and perpendicular to the sight line, on the basis of the voxel information stored in the voxel information storage section 10, and acquires a slice information group. This slice information group is at least temporarily stored in the volume rendering computer 13.

The above-mentioned slice information perpendicular to the sight line refers to a plane that is perpendicular to the sight line. For example, in a state in which the display 2 has been erected vertically, when it is viewed in a state in which it and the plane of the user's face are parallel, the slice information is in a plane perpendicular to the sight line.

The plurality of sets of slice information thus obtained include information about the points made up of $I(x,y,z,\alpha)$, as mentioned above. Thus, the slice information is such that a plurality of voxel labels 14 are disposed in the Z direction, for example. The group of voxel labels 14 is stored in the voxel label storage section 11.

Then, in S4, a rendered image is displayed on the display 2. At this point, the mouse 4 or the like is used to designate the range of CT values on the display 2, and the bone, blood vessel, or the like to be resected is selected and displayed.

Then, in S5, it is determined whether or not an instruction to perform registration has been received from the user. If a registration instruction has been received, the flow proceeds to A (S6) in order to perform registration. On the other hand, if a registration instruction has not been received, the flow proceeds to S7 to determine whether or not an instruction to perform navigation has been received.

If a registration instruction has been received in S5, registration is performed according to the flow shown in FIG. 7B.

Specifically, first, in S61, the position that will be the feature point of registration is given. More specifically, a portion of a bone whose position is easy to confirm from the body surface, such as the fifth spinous process and the left and right ilia, is used as the feature point.

Then, S62, while the surgeon, a nurse, etc., holds the sensor, it is pressed against a position near the feature point from the body surface of the patient lying on the operating table, and the position the sensor is finely adjusted while looking at the display 102 to acquire sensor position information.

Then, in S63, a conversion matrix for converting the real space coordinates indicating the acquired sensor position into virtual space coordinates is calculated.

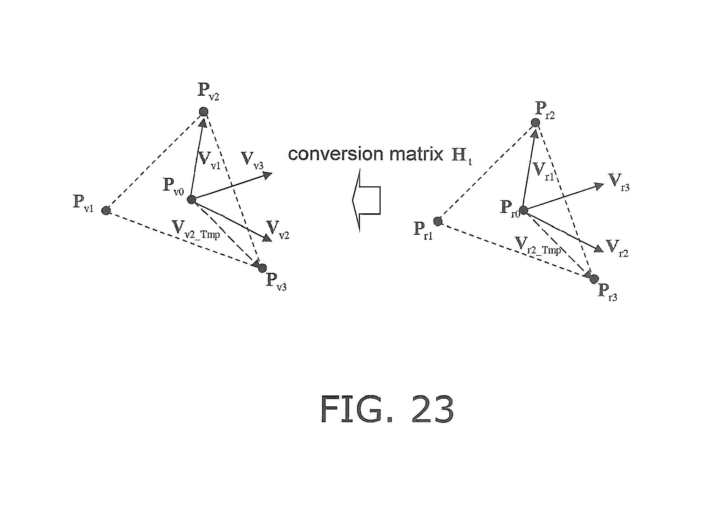

As shown in FIG. 23, the coordinate conversion matrix is found by the following procedure from three feature points ($P_{v1}$, $P_{v2}$, $P_{v3}$) designated in virtual space and $P_{v0}$ whose origin is a triangular center of gravity composed of the feature points ($P_{v1}$, $P_{v2}$, $P_{v3}$), and from feature point coordinates ($P_{r1}$, $P_{r2}$, $P_{r3}$) corresponding to an object in real space acquired from a sensor, and $P_{r0}$, whose origin is a triangular center of gravity composed of the feature point coordinates ($P_{r1}$, $P_{r2}$, $P_{r3}$).

First, since $P_{v0}$ is a feature point triangular center of gravity designated in virtual space, we obtain the following formula (1).

[First Mathematical Formula]

$$\mathbf{P}_{v0} = \frac{(\mathbf{P}_{v1} + \mathbf{P}_{v2} + \mathbf{P}_{v3})}{3} \tag{1}$$

The orthonormal vector in virtual space is found by the following procedure from this virtual space origin vector $\mathbf{P}_{v0}$ and the three feature points $\mathbf{P}_{v1}$, $\mathbf{P}_{v2}$, and $\mathbf{P}_{v3}$.
A uniaxial vector $V_{v1}$ is defined by the following formula (2),

[Second Mathematical Formula]

$$\mathbf{V}_{v1} = \frac{1}{|\mathbf{P}_{v2} - \mathbf{P}_{v0}|}(\mathbf{P}_{v2} - \mathbf{P}_{v0}) \tag{2}$$

a temporary biaxial vector $V_{v2\_Tmp}$ for finding a vector perpendicular to a plane including the feature points $P_{v2}$ and $P_{v3}$ as a third axis defined by the following formula (3),

[Third Mathematical Formula]

$$\mathbf{V}_{v2\_Tmp} = \frac{1}{|\mathbf{P}_{v3} - \mathbf{P}_{v0}|}(\mathbf{P}_{v3} - \mathbf{P}_{v0}) \tag{3}$$

a triaxial vector $V_{v3}$ is found by taking the cross product of $V_{v1}$ and $V_{v2\_Tmp}$, and

[Fourth Mathematical Formula]

$$\mathbf{V}_{v3} = \mathbf{V}_{v1} \times \mathbf{V}_{v2\_Tmp} \tag{4}$$

a biaxial vector $V_{v2}$ is found by taking the cross product of $V_{v3}$ and $V_{v1}$.

[Fifth Mathematical Formula]

$$\mathbf{V}_{v2} = \mathbf{V}_{v3} \times \mathbf{V}_{v1} \tag{5}$$

By the same procedure, $P_{r0}$ is found from a real space feature point triangular center of gravity:

[Sixth Mathematical Formula]

$$\mathbf{P}_{r0} = \frac{(\mathbf{P}_{r1} + \mathbf{P}_{r2} + \mathbf{P}_{r3})}{3} \qquad (6)$$

and the orthonormal vectors $V_{r1}$, $V_{r2}$, and $V_{r3}$ of real space are found as follows from $P_{r0}$ and the three feature points $P_{r1}$, $P_{r2}$, and $P_{r3}$.

[Seventh Mathematical Formula]

$$\mathbf{V}_{r1} = \frac{1}{\left|\mathbf{P}_{r2} - \mathbf{P}_{r0}\right|}\left(\mathbf{P}_{r2} - \mathbf{P}_{r0}\right) \qquad (7)$$

[Eighth Mathematical Formula]

$$\mathbf{V}_{r2\_Tmp} = \frac{1}{\left|\mathbf{P}_{r3} - \mathbf{P}_{r0}\right|}\left(\mathbf{P}_{r3} - \mathbf{P}_{r0}\right) \qquad (8)$$

[Ninth Mathematical Formula]

$$\mathbf{V}_{r3} = \mathbf{V}_{r1} \times \mathbf{V}_{r2\_Tmp} \qquad (9)$$

[Tenth Mathematical Formula]

$$\mathbf{V}_{r2} = \mathbf{V}_{r3} \times \mathbf{V}_{r1} \qquad (10)$$

Next, a rotation matrix to each of the spatial coordinates is found from virtual space and real space orthonormal vectors. First, a rotation matrix $M_v$ in virtual space is as follows,

[Eleventh Mathematical Formula]

$$\mathbf{M}_{\mathrm{v}} = \begin{bmatrix} \mathbf{V}_{v1} & \mathbf{V}_{v2} & \mathbf{V}_{v3} \end{bmatrix}^{T} \qquad (11)$$

and a rotation matrix $M_r$ in real space is as follows,

[Twelfth Mathematical Formula]

$$\mathbf{M}_{\mathrm{r}} = \begin{bmatrix} \mathbf{V}_{r1} & \mathbf{V}_{r2} & \mathbf{V}_{r3} \end{bmatrix}^{T} \qquad (12)$$

In order to find a rotation matrix from a real space coordinate system to a virtual space coordinate system, a rotation matrix from a real space coordinate system to a real space coordinate system is necessary. This is an inverse matrix since the conversion is the inverse of that produced with a rotation matrix of a real space coordinate system. A real space coordinate system converted by this inverse matrix is subjected to conversion by a rotation matrix of a virtual space coordinate system, which gives a rotation matrix $M_{rotate}$ from a real space coordinate system to a virtual space coordinate system. Expressed as an equation, this gives the following formula (13).

[Thirteenth Mathematical Formula]

$$\mathbf{M}_{\mathrm{rotate}} = \mathbf{M}_{\mathrm{v}} \mathbf{M}_{\mathrm{r}}^{-1} \qquad (13)$$

With a scaling matrix $H_{scale}$, the DICOM data is believed to be the same as in a real space, so the same applies to a virtual space. Thus, this is defined as a unit matrix.

The rotation matrix $M_{rotate}$ thus found, and the virtual space origin $P_{v0}$, which is the average movement section with a scaling matrix, give the following conversion matrix $H_t$ from a real space coordinate system to a virtual space coordinate system.

[Fourteenth Mathematical Formula]

$$\mathbf{H}_{\mathrm{t}} = \begin{pmatrix} \mathbf{H}_{\mathrm{scale}} \mathbf{M}_{\mathrm{rotate}} & \mathbf{P}_{v0} \\ \mathbf{0} & 1 \end{pmatrix} \qquad (14)$$

In this embodiment, this conversion matrix is used to convert the real space coordinates acquired from the three-dimensional sensor 32a into virtual space coordinates.

A plurality of these conversion matrixes H are kept in the conversion matrix holder 28.

Then, in S64, it is determined whether or not the registration is sufficiently accurate. At this point steps S61 to S64 are repeated until it can be confirmed that the registration accuracy is within a predetermined range. Processing is ended at the stage when accuracy has been confirmed to be within a specific range.

That is, in S64, if it is found that the registration accuracy is not within a predetermined range, registration is performed again to correct the first result. This allows the accuracy of the registration processing to be improved.

Registration correction processing will be discussed in detail below.

As discussed above, when an instruction to carry out registration has been received in S5, the flow proceeds directly to S7 after the registration is performed, or if no instruction to carry out registration has been received.

Then, in S7, if an instruction to carry out navigation during surgery has been received, the flow proceeds to B (S8). On the other hand, if an instruction to carry out navigation has not been received, the flow returns to the processing of S3.

Specifically, in S81, the endoscope/surgical instrument position and attitude acquisition section 26 acquires the three-dimensional positions of the oblique endoscope 32 and the surgical instrument 33 on the basis of the sensing result of the position and angle sensing device 29.

Then, in S82, the above-mentioned conversion matrix H is used to convert from a real space coordinate system to a virtual space coordinate system on the basis of the three-dimensional positions of the oblique endoscope 32 and the surgical instrument 33.

Then, in S83, the volume rendering computer 13 acquires endoscope parameters from the endoscope parameter storage section 22.

Then, in S84, the volume rendering computer 13 acquires surgical instrument parameters from the surgical instrument parameter storage section 24.

Then, in S85, endoscope video is acquired from the endoscope video acquisition section 30.

Then, in S86, if a plurality of sites are to be resected, it is confirmed whether or not computation of the distance from the distal end of the surgical instrument 33 to all of the resection sites has been completed. If this distance computation has been completed, the flow proceeds to S87.

Then, in S87, the volume rendering computer 13 displays a three-dimensional image (rendered image) on the displays 2 and 102, superposed with the endoscope video.

At this point, the three-dimensional sensor 33b senses the movement of the actual surgical instrument 33, and the movement of the surgical instrument 33 is displayed in real time on the three-dimensional image, which allows the surgeon to manipulate the surgical instrument 33 while checking distance information displayed on the display 102. This allows surgery navigation that is useful to the surgeon to be carried out.

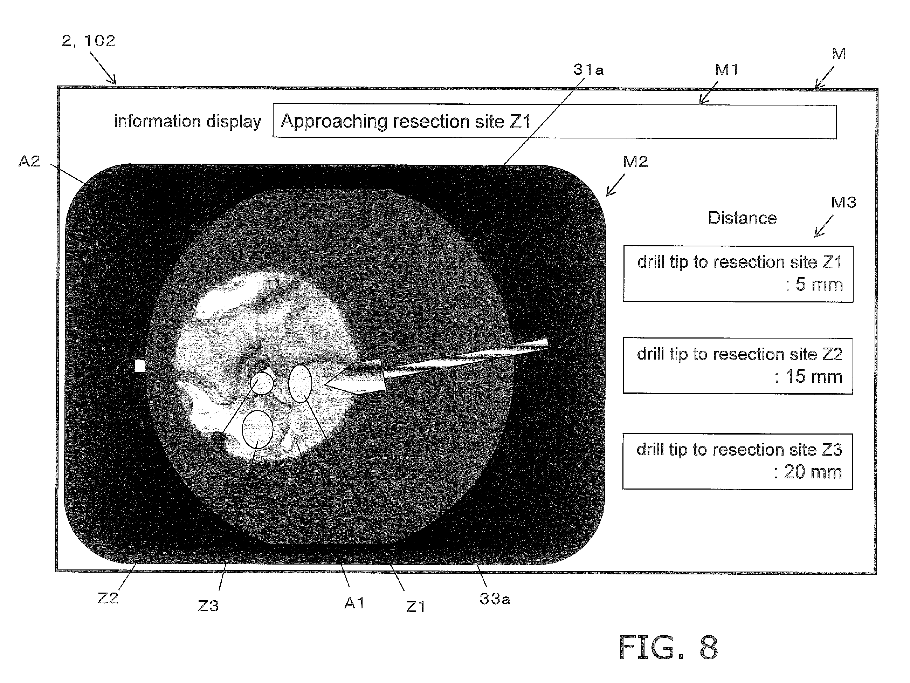

The three-dimensional image displayed on the displays 2 and 102 in S87 will now be described through reference to FIGS. 8 to 12.

In the example shown in FIGS. 8 to 12, there are three resection sites Z1 to Z3, but a case in which resection is performed by moving the surgical instrument 33 closer to the resection site Z1 will be described.

Specifically, as shown in FIG. 8, the monitor screen M of the displays 2 and 102 includes an information display area M1, a navigation image area M2, and a distance display area M3 as navigation screens.

More specifically, text information consisting of "Approaching resection site" is displayed in the information display area M1. An image obtained by superposing the surgical instrument image 33a, a retractor image 31a, and the resection sites Z1 to Z3 over a three-dimensional image of the area around the resection site is displayed in the navigation image area M2. The distance from the multiple points for the distal end of the drill (surgical instrument 33) to the various resection sites Z1 to Z3 is displayed in the distance display area M3.

Regarding the superposition of the various images in the navigation image area M2, the transmissivity can be set for each image, and changed so that information that is important to the surgeon will be displayed.

As shown in FIG. 9, when the speed at which the surgeon moves the surgical instrument 33 toward the resection site Z1 is increased in order to resect the resection site Z1, a message of "Approaching the resection site Z1. Approach speed is gradually increasing" is displayed in the information display area M1. The information display area M1 here is displayed with a yellow background, for example, in order to warn the surgeon.

Also, when the speed at which the surgeon moves the surgical instrument 33 toward the resection site Z1 is increased, there is the risk that the approach speed of the surgical instrument 33 will be too high, causing the surgical instrument to pass by the portion to be resected. In view of this, in this embodiment, as shown in FIG. 10, a message of "Approaching resection site Z1. Approach speed is too high" is displayed in the information display area M1. The information display area M1 here is displayed with a red background, for example, in order to give a more serious warning to the surgeon.

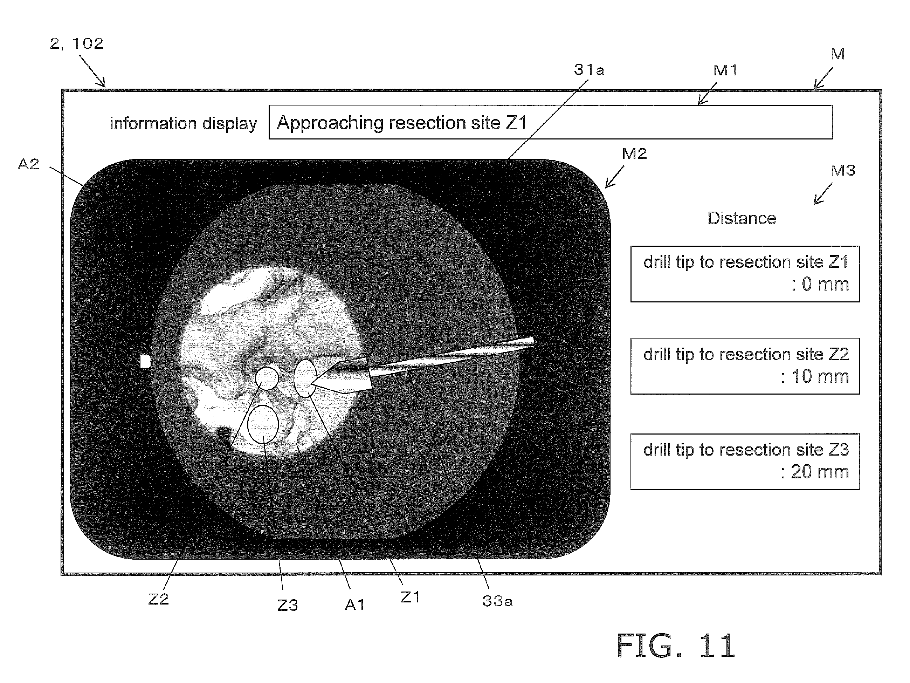

Next, when the surgeon moves the resection site Z1 toward the resection site Z1 in order to resect the resection site Z1, as shown in FIG. 11, the distal end portion of the surgical instrument image 33a is displayed in a state of being in contact with the resection site Z1 in the navigation image area M2. The distance display area M3 here displays that the distance is 0 mm from the drill tip to the resection site Z1.

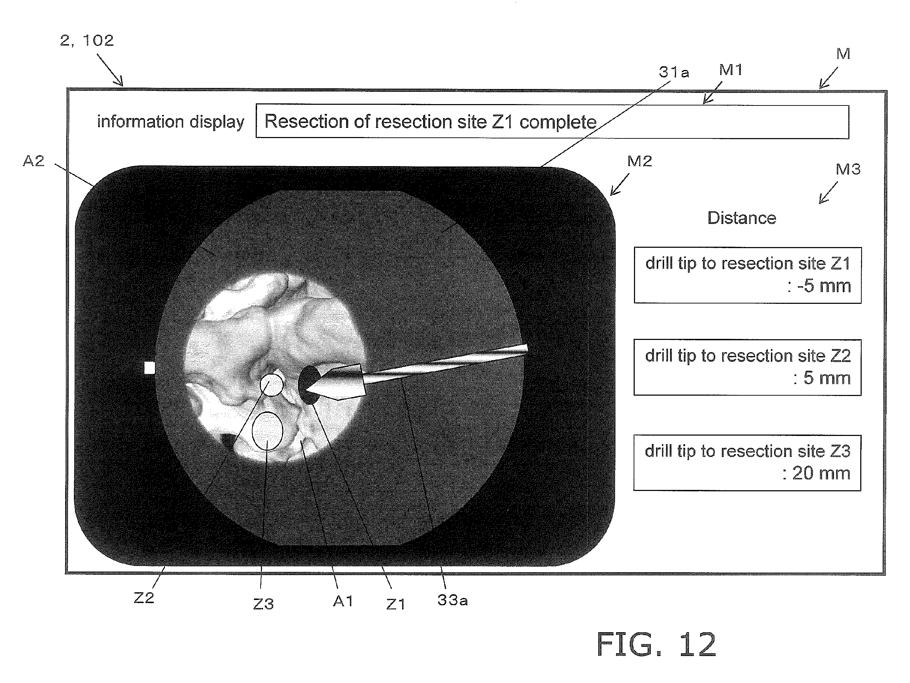

Next, when the surgical instrument 33 is used to resect the resection site Z1, as shown in FIG. 12, the navigation image area M2 displays that the distal end portion of the surgical instrument image 33a is moving into the resection site Z1. At this point, for example, if the surgical instrument 33 resects to a depth of 5 mm, the distance display area M3 displays that the distance from the drill tip to the resection site Z1 is -5 mm. A message of "Resection of resection site Z1 complete" is displayed in the information display area M1.

As described above, the personal computer 1 of the surgery assistance device 100 in this embodiment converts the actual three-dimensional position (real space coordinates) of the oblique endoscope 32 or the surgical instrument 33 into coordinates (virtual space coordinates) on a three-dimensional image produced by the volume rendering computer 13, and then performs navigation during surgery while displaying a combination of an image indicating the distal end of the surgical instrument 33 (the surgical instrument image 33a) and the distance from the surgical instrument distal end to the resection site into a three-dimensional image.

This allows the surgeon to manipulate the surgical instrument 33 while confirming the distance from the distal end of the surgical instrument 33 to the resection site Z1, and while looking at the screen of the display 102.

Method for Displaying Retractor Image in Navigation Image Area

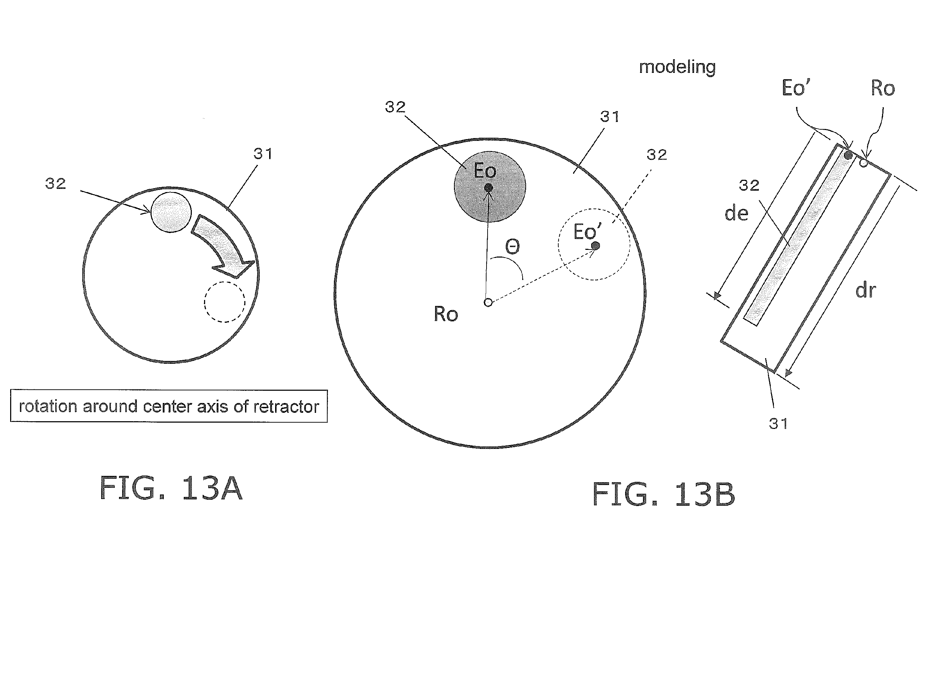

Next, FIGS. 13A and 13B will be used to describe mapping from two-dimensional input with the mouse 4 to three-dimensional operation with the endoscope 3.

Here, the display on the three-dimensional image is made on the basis of parameters such as the diameter, length, and movement direction (insertion direction) of the retractor, and the result of measuring the position and attitude with the sensor installed in the retractor.

Usually, the oblique endoscope 32 (see FIG. 13A, etc.) inserted into the retractor 31 is fixed to an attachment (not shown) that is integrated with the retractor 31, which limits movement in the peripheral direction within the retractor 31.

As shown in FIG. 13A, assuming that the oblique endoscope 32 has been rotated along with the attachment, the rotation matrix $R\Theta$ after rotation by an angle $\Theta$ is calculated with respect to the axis Rz in the depth direction of the distance Ro from the center of the retractor 31 to the center of the oblique endoscope 32.

Next, since the vector RoEo' = $R\Theta \times$ RoEo, the endoscope distal end position can be calculated from the equation of the endoscope distal end position Ec = Eo' + Rz*de, using the insertion depth de of the endoscope.

This allows the three-dimensional endoscope distal end position to be calculated by two-dimensional mouse operation.

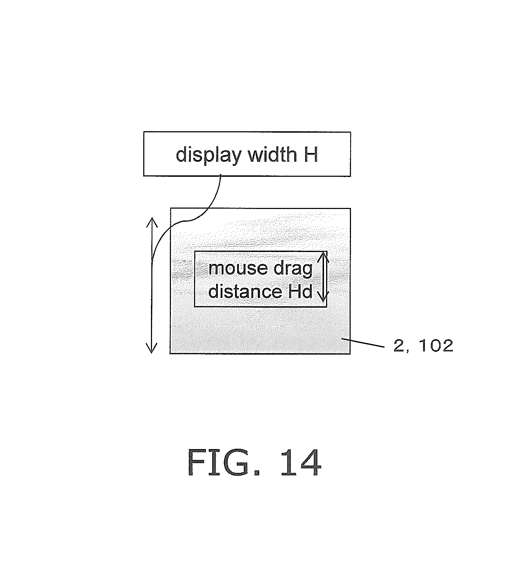

Next, another example related to mapping from two-dimensional input with the mouse 4 to three-dimensional operation with the endoscope 3 will be described through reference to FIG. 14.

An endoscope is usually connected to the rear end side of a camera head that houses a CCD camera (not shown). The rotation of the display when this camera head is rotated will now be described.

Specifically, in actual endoscopic surgery, if the image displayed on the display screens of the displays 2 and 102 ends up being displayed vertically, just the image is rotated, without changing the field of view, by rotating the camera head in order to align the orientation of the actual patient with the orientation of the display on the displays 2 and 102.

In order to achieve this by two-dimensional input using the mouse 4, first, the $\Theta$ = 360*Hd/H is calculated from the mouse drag distance and the display height.

Then, the rotation matrix $R2\Theta$ after rotation of an angle $\Theta$ is calculated with respect to the axis Ry in the depth direction of the screen center coordinates of the displays 2 and 102.

Then, the image displayed on the displays 2 and 102 can be rotated 90 degrees, without changing the field of view, by using U' = $R2\Theta$*U as the new upward vector for the upward vector U of the field of view.

Consequently, an image displayed on the displays 2 and 102 can be easily adjusted to the same orientation (angle) as the monitor screen in actual endoscopic surgery by two-dimensional input with the mouse 4.

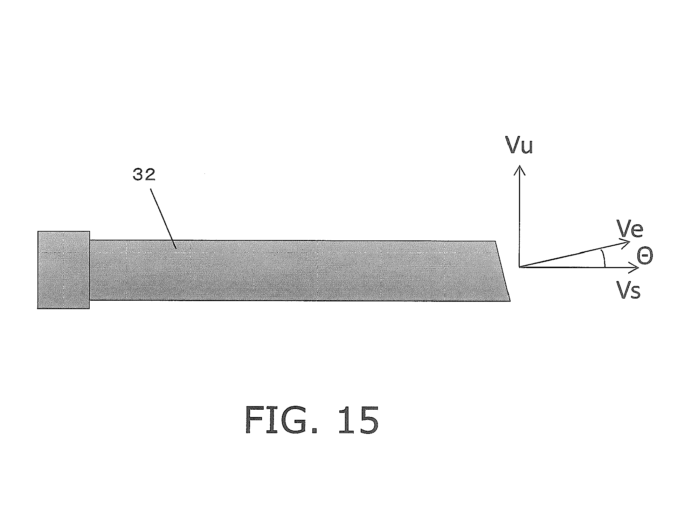

Next, the method for producing a volume rendering image that reflects any oblique angle of the oblique endoscope 32 will be described through reference to FIG. 15.

Specifically, in this embodiment, a rotation matrix is applied to the field vector according to the oblique angle set for each oblique endoscope 32.

More specifically, first the cross product Vc of the vertical vector Vu corresponding to the perspective direction of the oblique endoscope 32 and the endoscope axis vector Vs corresponding to the axial direction of the retractor 31 are calculated.

Then, the rotation matrix Rs that undergoes $\Theta$ rotation around the Vc is calculated.

Then, the field vector Ve that reflects the oblique angle can be found as Ve = Rs*Vs.

Consequently, even if the oblique angle is different for each oblique endoscope 32, the field of view range can be set for each oblique endoscope 32 used in surgery by calculating the field vector Ve on the basis of the information stored in the endoscope parameter storage section 22, etc.

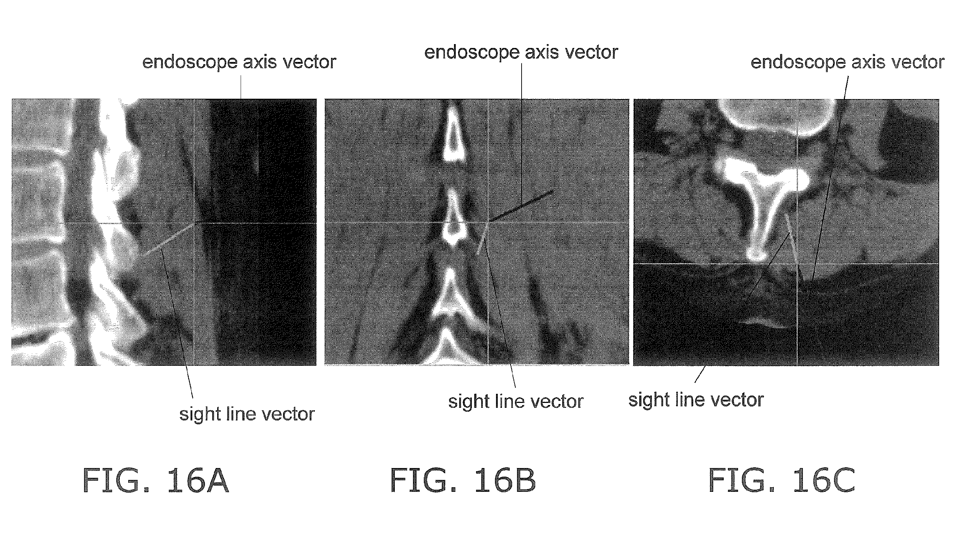

FIGS. 16A to 16C show the state when the endoscope axis vector Vs and the field vector Ve are used to show the distal end position of the oblique endoscope 32 and the field vector in a three-panel view.

As shown in FIGS. 16A to 16C, this allows the insertion direction of the oblique endoscope 32 to be easily ascertained by using a front view (as seen from the side of the patient), a plan view (as seen from the back of the patient), and a side view (as seen from the spine direction of the patient) in a simulation of surgery for lumbar spinal stenosis using the oblique endoscope 32.

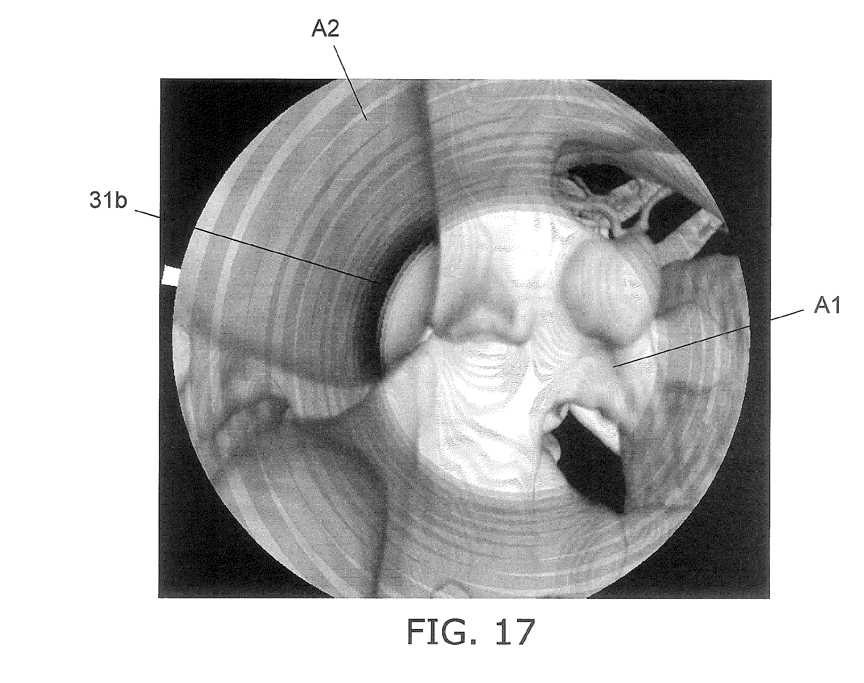

With the personal computer 1 in this embodiment, because of the above configuration, an endoscopic image (the endoscopic image display area A1) that shows the restricted display area A2 that is blocked by the retractor 31 is displayed as shown in FIG. 17 in an endoscopic surgery simulation, on the basis of the shape of the retractor 31, the oblique angle and view angle of the oblique endoscope 32, and so forth.

Consequently, a display that approximates the image displayed on the display screen in an actual endoscopic surgery can be displayed by creating a display state that shows the restricted display area A2, which cannot be seen because it is behind the inner wall of the retractor 31 in an actual endoscopic surgery. Therefore, surgery can be assisted more effectively.

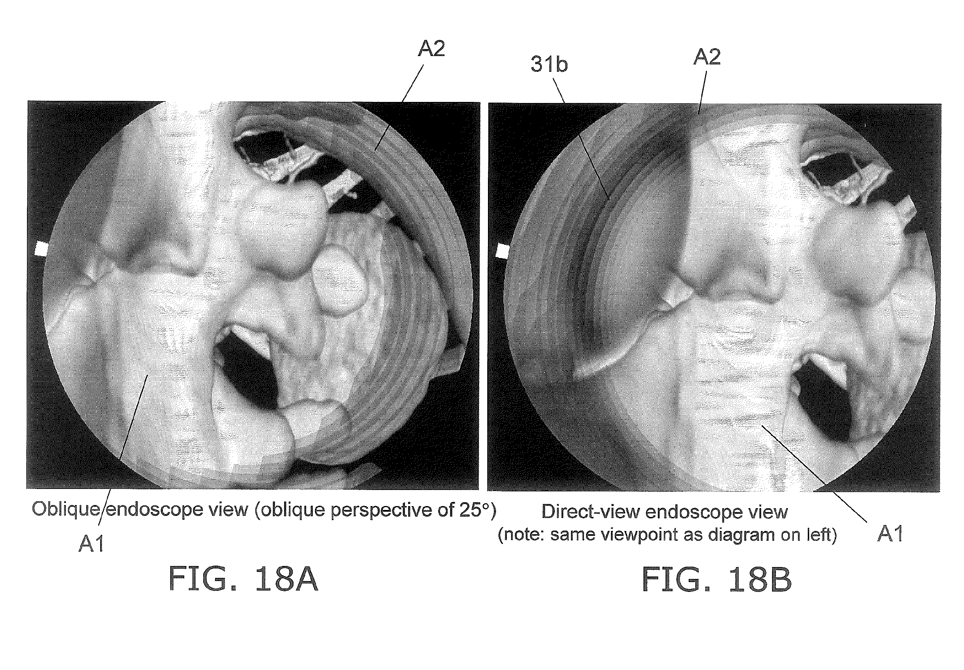

As shown in FIG. 18A, if the oblique angle of the oblique endoscope 32 is 25 degrees, for example, the surgical site will be displayed within the endoscope display area A1 by showing the restricted display area A2 produced by the retractor 31.

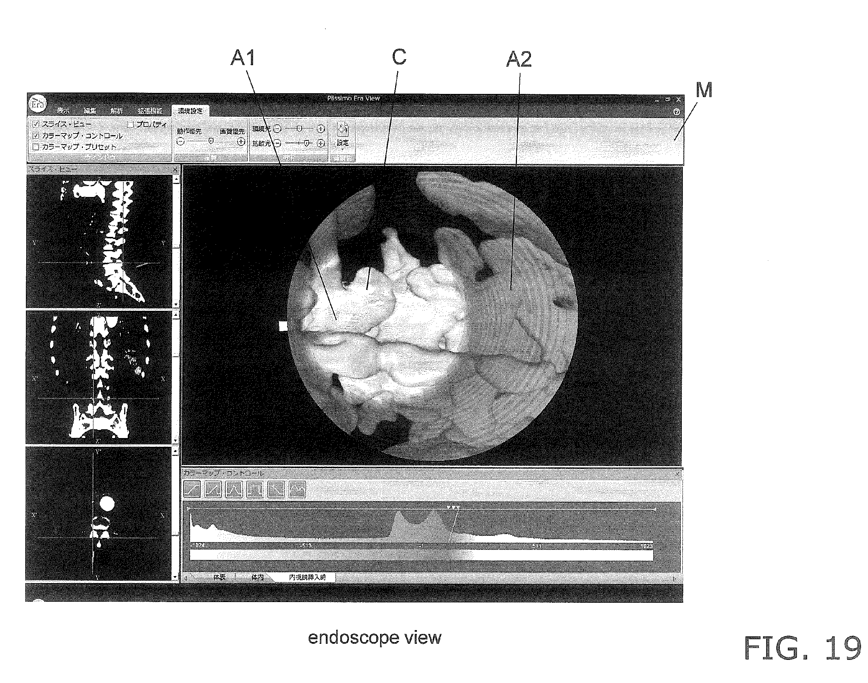

Furthermore, as shown in FIG. 19, the image that is actually displayed on the displays 2 and 102 of the personal computer 1 in this embodiment can also be combined with the display of a resection target site C or the like, for example, allowing the restricted display area A2 to be shown while displaying the resection target site C within the endoscope display area A1.

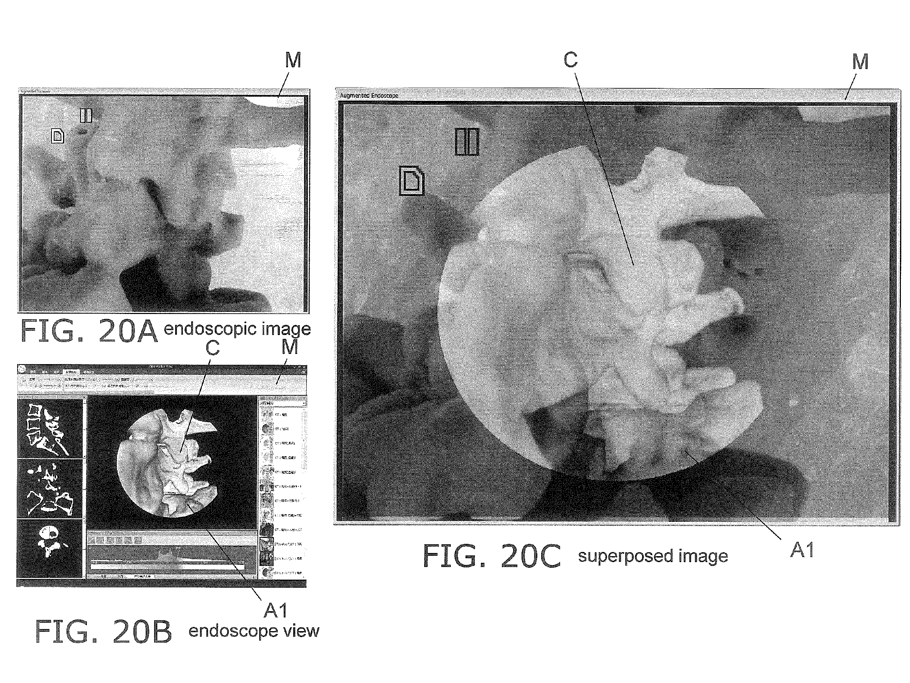

Further, in order to display a navigation screen that is easy for the surgeon to understand, as shown in FIGS. 20A to 20C, an endoscope image centered on the cutting target site C, an endoscope view cropped from the three-dimensional image corresponding to this portion, and an image in which the endoscopic image and the endoscope view are superposed may each be displayed on the monitor screen M.

With the superposed image in FIG. 20C, a case is shown in which the transmissivity of the endoscope view has been set to 30%. The transmissivity of the endoscope view can be set as desired between 0 and 100%.

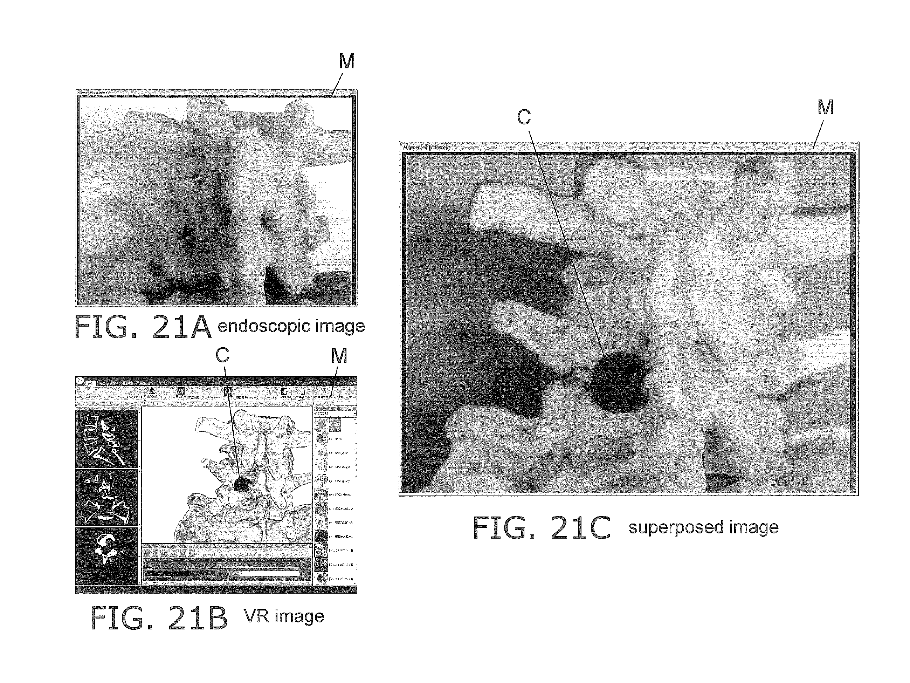

Also, the three-dimensional image that is combined with the endoscopic image is not limited to being an endoscope view. For example, as shown in FIGS. 21A to 21C, a VR image showing an endoscope image to be centered on the cutting target site C, a three-dimensional image corresponding to that portion, and an image in which the endoscopic image and the endoscope view are superposed may each be displayed on the monitor screen M.

With the superposed image in FIG. 21C, the transmissivity of the VR image is set to 50%.

Registration Processing

With the surgery assistance system 100 in this embodiment, as described above, registration, in which the positions are matched between real space coordinates and virtual space coordinates, is performed before surgical navigation is carried out. This registration will now be described in greater detail.

In this embodiment, registration of the real space coordinates and virtual space coordinates (three-dimensional image coordinates) is carried out as follows.

The registration function here finds the positional relation to the most important part of the oblique endoscope 32 during surgery, so it is a function for positioning between the virtual space coordinates had by the three-dimensional image and the real space coordinates indicating position information from the three-dimensional sensor 32a attached on the endoscope 32 side. This registration function makes it possible to acquire the position of the endoscope 32 in virtual space by using a coordinate conversion matrix produced in the course of processing of this registration, and to interactively perform volume rendering that reflects the final fisheye characteristics.

In the positioning of the various coordinates, three of the feature points corresponding to within real space and three of the feature points corresponding to within virtual space are defined, the amount of scaling, the amount of parallel movement, and the amount of rotation are calculated from these coordinates, and the final coordinate conversion matrix is created.

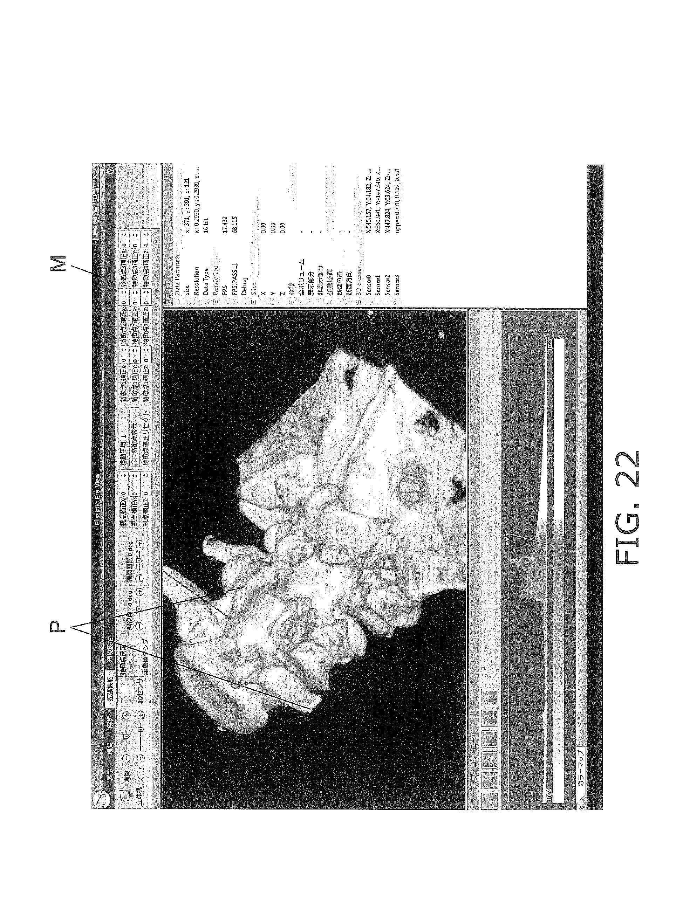

FIG. 22 shows the monitor screen M displaying a registration-use interface screen for setting feature points (the points P in the drawing).

The flow of registration will now be described.

First, three feature point coordinates $(x_v, y_v, z_v)$ are defined (the converted coordinate values are in the same mm units as the coordinates acquired by sensor) in virtual space sampled with a mouse, with respect to the three-dimensional image displayed in the view window.

Next, the corresponding feature point coordinates $(x_r, y_r, z_r)$ are pointed to with a magnetic sensor and registered in order, with respect to an object in real space. The feature point position information defined in two spaces is used to calculate the origins, thereby calculating the vector of parallel movement.

Next, the scaling matrix and the rotation matrix are calculated, and the final coordinate conversion matrix is put together and stored.

Also, with an oblique endoscope, it is necessary to sense not only the position of the endoscope distal end, but also the orientation of the endoscope axis, and since the rotation matrix produced during the above-mentioned computation is used in calculating the field of view in virtual space, the rotation matrix is also stored by itself.

Registration Correction

As shown in FIG. 7B, in this embodiment, when registration is performed, the accuracy of the registration is confirmed in S64.

Here, after the registration has been performed, if there is more than a specific amount of deviation in the feature point position designation in real space corresponding to the feature points in virtual space, the following processing is carried out to correct this.

Specifically, the personal computer 1 in this embodiment has a correction function for correcting deviation with an interface while confirming the coordinate axes and the deviation in feature points displayed on a volume rendering image in virtual space.

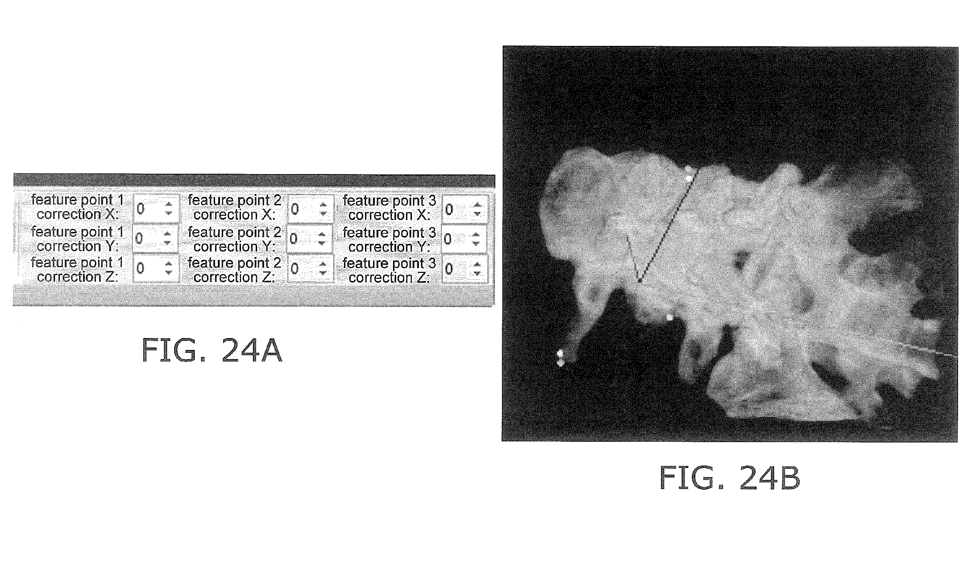

FIGS. 24A and 24B show an example of displaying coordinate axes and feature points on a volume rendering image and a correction value setting interface.

The flow in registration correction using this correction function is as follows.

when the user sets a feature point correction value within the interface shown in FIG. 24A, coordinate correction by vector summing is performed on the feature points in the registered real space, and registration processing is performed again.

In this re-registration, just as with the registration function, the feature point coordinates defined in two spaces are used to perform recalculation of the rotation matrix and the coordinate conversion matrix.

When this recalculation is finished, the positions where the feature points and the coordinate axes are to be drawn are recalculated, and the volume rendering image is updated as shown in FIG. 24B.

Equistance Display Control Centered on Resection Site

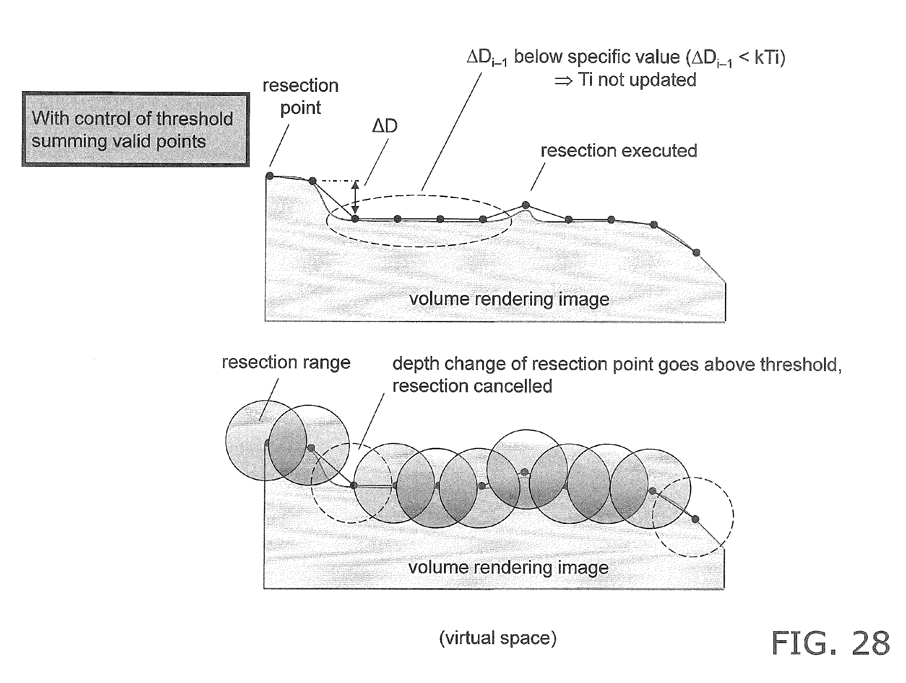

In this embodiment, as shown in FIG. 28, for example, the screen showing the virtual space actually displayed on the displays 2 and 102 of the personal computer 1 can be set to a distance $l_1$ region and a distance $l_2$ region centered on the resection site, and these regions can be displayed in different colors.

This makes it easy for the surgeon to tell how far it is from the distal end of the surgical instrument 33 to the resection site.

Resection Restriction in Resection Simulation

In this embodiment, when a resection simulation is performed prior to surgery, the depth controller 17 computes the change in depth or discontinuity around the resection site on the basis of the depth position of the resection site sensed by the depth sensor 15.

If the extent of this change exceeds a specific threshold, the voxel label setting section 18 and the resected voxel label calculation display section 19 perform control so that resection is halted in the virtual space used for simulation, or the resection data is not updated.

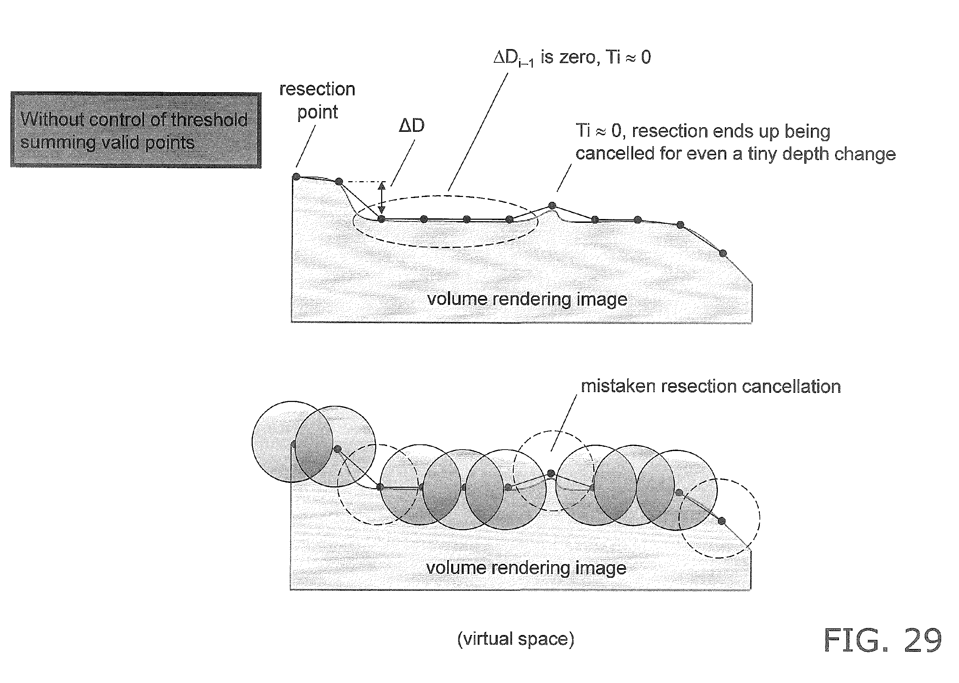

More specifically, as shown in FIG. 29, when the concept of threshold summing valid points is used to perform a resection simulation in which the resection goes from a resection point progressively to the right, if the amount of change in depth (depth change amount) $\Delta D$ is over a specific threshold, no resection will be performed at that resection point in the volume rendering image in virtual space.

Specifically, when the concept of threshold summing valid points is introduced, if the depth change from the immediately prior threshold summing valid point is below a specific value with respect to a resection point i-1, it is not treated as a new threshold summing valid point, so even if resection is continued in a flat plane, a restriction can be imposed so that $T_i$ does not contract to zero.

[Fifteenth Mathematical Formula]

$$\mathbf{T}_i = \begin{cases} \mathbf{T}_{i-1} & if\ \Delta\mathrm{D}_{i-1} < k\mathbf{T}_{i-1} \\ m\left(\displaystyle\sum_{k=i-1-n}^{k=i-1}\Delta\mathrm{D}_k\right)/n & if\ \Delta\mathrm{D}_{i-1} \ge k\mathbf{T}_{i-1} \end{cases} \qquad (15)$$

$\Delta D_k$: depth change from immediately prior threshold summing valid point at threshold summing valid point k
m: resectable point evaluation coefficient (at least 1.0)
k: threshold summing valid point evaluation coefficient (at least 0.0 and less than 1.0)

In the above formula, if $\Delta D_{i-1} < kT_{i-1}$ is true, the resection point i-1 is not treated as a new threshold summing valid point, and $T_i = T_{i-1}$. Otherwise, it is treated as threshold to be added as with a conventional method.

Thus, if the resection point moves through a relatively flat portion where the depth change amount $\Delta D_{i-1}$ is less than a specific value ($\Delta D_{i-1} < kTi$), the resection simulation is performed so as not to update Ti.

Consequently, in a portion where the depth position changes greatly, either the resection data is not updated, or resection is halted, which allows the proper resection simulation image to be displayed.

Meanwhile, if the above-mentioned control of the threshold summing valid points is not performed, as shown in FIG. 29, when resection simulation is carried out in which the resection goes from a certain resection point progressively to the right, if the amount of change in depth (depth change amount) $\Delta D$ is over a specific threshold, then just as in FIG. 28, no resection will be performed at that resection point in the volume rendering image in virtual space.

However, if the above-mentioned control of the threshold summing valid points is not performed, $\Delta D_{i-1}$ will be zero, and Ti $\approx$ 0, in a relatively flat portion, so even a tiny depth change can create a problem that leads to the cancellation of resection.

Thus, in this embodiment, when resection simulation is performed by using the above-mentioned concept of threshold summing valid points, the resulting display will be close to the intended resection simulation image.

Other Embodiments

An embodiment of the present invention was described above, but the present invention is not limited to or by the above embodiment, and various modifications are possible without departing from the gist of the invention.

(A) In the above embodiment, an example was described in which the present invention was in the form of a surgery assistance device, but the present invention is not limited to this.

For example, the present invention can be in the form of a surgery assistance program that allows a computer to execute the control method shown in FIGS. 7A to 7C

(B) In the above embodiment, an example was described in which a single three-dimensional sensor 32a, which is a six-axis sensor, was attached to the oblique endoscope 32 in order to sense the three-dimensional position and attitude of the oblique endoscope 32 or the surgical instrument 33, but the present invention is not limited to this.

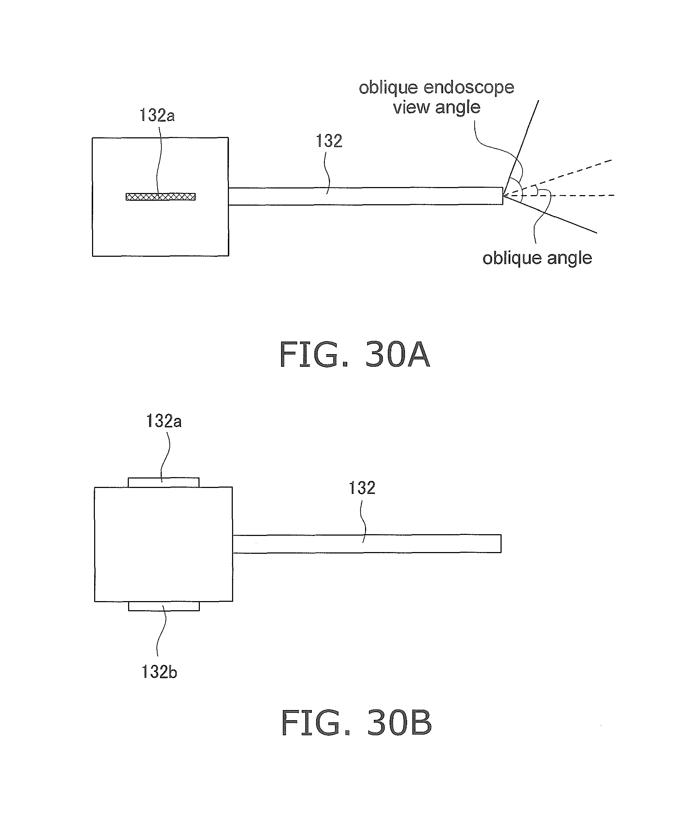

As shown in FIGS. 30A and 30B, for example, two three-dimensional sensors 132a and 132b, which are fix-axis sensors, may be attached to an endoscope 132.

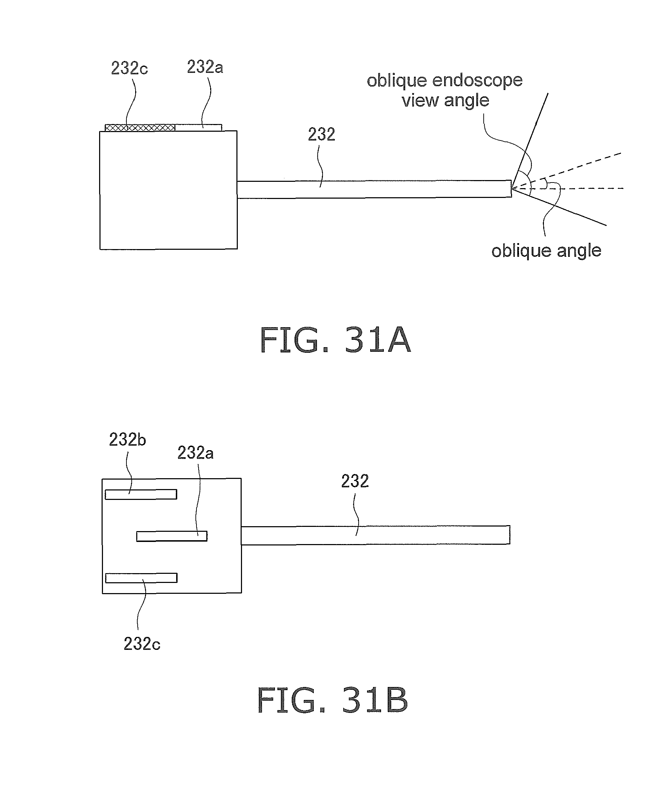

Furthermore, as shown in FIGS. 31A and 31B, for example, three three-dimensional sensors 232a, 232b, and 232c, which are three-axis sensors, may be attached to the endoscope 232.

(C) In the above embodiment, an example was given in which the six-axis sensor 32a was attached near the rear end of the oblique endoscope 32 in order to sense the three-dimensional position and attitude of the oblique endoscope 32 or the surgical instrument 33, but the present invention is not limited to this.

For example, the position where the three-dimensional sensor is attached is not limited to being near the rear end of the endoscope or surgical instrument, and may instead be near the center or the distal end side.

INDUSTRIAL APPLICABILITY

The surgery assistance device of the present invention has the effect of allowing the proper navigation to be performed during surgery while the user looks at the resection site to be resected with the surgical instrument, and therefore can be widely applied as a surgery assistance device in performing various kinds of surgery.

REFERENCE SIGNS LIST

1 personal computer (surgery assistance device)
2 display (display component)
2a endoscopic image display monitor
2b three-dimensional image display monitor
3 keyboard (input component)
4 mouse (input component)
5 tablet (input component)
6 tomographic image information acquisition section
7 voxel information extractor

8 tomographic image information section
9 memory
10 voxel information storage section
11 voxel label storage section
12 color information storage section
13 volume rendering computer (distance calculator, simulator, navigator)
15 depth sensor (simulator)
16 bus
17 depth controller (simulator)
18 voxel label setting section (simulator)
19 resected voxel label calculation display section (simulator)
20 window coordinate acquisition section
21 color information setting section
22 endoscope parameter storage section
23 endoscope parameter setting section
24 surgical instrument parameter storage section
25 surgical instrument parameter setting section
26 endoscope/surgical instrument position and attitude acquisition section (endoscope/surgical instrument position sensor)
27 registration computer
28 conversion matrix holder
29 position and angle sensing device
30 endoscope video acquisition section
31 retractor
31 a retractor image
31b collision site
32 oblique endoscope (endoscope)
32a six-axis sensor
33 surgical instrument
33a surgical instrument image
33b three-dimensional sensor
34 box-type transmitter (magnetic field generator)
100 surgery assistance system
102 liquid crystal display (display component)
132 endoscope
132a, 132b five-axis sensor
232 endoscope
232a, 232b, 232c three-axis sensor
A1 endoscopic image display area (first display area)
A2 restricted display area (second display area)
C resection site
M monitor screen
M1 information display area
M2 navigation image area
M3 distance display area
Z1 to Z3 resection site

**Claims**

1. A surgery assistance device configured to perform navigation while displaying a three-dimensional simulation image produced from tomographic image information during surgery in which a resection-use surgical instrument is used while the user views an endoscopic image, the device comprising:

a tomographic image information acquisition section configured to acquire tomographic image information about a patient;
a memory that is connected to the tomographic image information acquisition section and configured to store voxel information for the tomographic image information;

a volume rendering computer that is connected to the memory and configured to sample voxel information in a direction perpendicular to the sight line on the basis of the voxel information;

an endoscope/surgical instrument position sensor configured to sequentially sense the three-dimensional positions of the endoscope and the surgical instrument;

a registration computer configure to integrate the coordinates of a three-dimensional image produced by the volume rendering computer and the coordinates of the endoscope and the surgical instrument sensed by the endoscope/surgical instrument position sensor;

a simulator configured to store the resection portion scheduled for surgery and virtually resected on the three-dimensional image produced by the volume rendering computer, in the memory after associating it with the voxel information;

a distance calculator configured to calculate a distance between the working end of the surgical instrument on the three-dimensional image and the voxel information indicating the resection portion and stored in the memory; and

a navigator configured to display the working end of the surgical instrument on the three-dimensional image by using the coordinates of the surgical instrument during surgery, and display the distance between the working end and the voxel information indicating the resection portion stored in the memory, along with the endoscopic image displayed during surgery.

2. The surgery assistance device according to Claim 1,
wherein the simulator senses the depth of the surgical site during pre-surgery resection and computes the degree of change in depth or discontinuity, and stops the resection or does not update the resection data if the degree of change exceeds a specific threshold.

3. The surgery assistance device according to Claim 1 or 2,
wherein the navigator models, by multi-point model, the working end of the surgical instrument on the three-dimensional image.

4. The surgery assistance device according to any of Claims 1 to 3,
wherein the navigator uses a vector that has a component of the direction of voxel information indicating the resected portion by the surgical instrument during surgery as the vector of the distance.

5. The surgery assistance device according to any of Claims 1 to 4,
wherein the navigator changes the display color of the voxels for each equidistance from the resection portion.

6. The surgery assistance device according to any of Claims 1 to 5,
wherein, after integrating the coordinates of a three-dimensional image and the coordinates of the endoscope and the surgical instrument, the registration computer checks the accuracy of this coordinate integration, and corrects deviation in the coordinate integration if this accuracy exceeds a specific range.

7. The surgery assistance device according to any of Claims 1 to 6,
wherein the navigator sets and displays a first display area acquired by the endoscope and produced by the volume rendering computer, and a second display area in which the display is restricted by the surgical instrument during actual surgery.

8. The surgery assistance device according to any of Claims 1 to 7,
further comprising a display section that displays the three-dimensional image, an image of the distal end of the surgical instrument, and the distance.

9. A surgery assistance program configured to perform navigation while displaying a three-dimensional simulation image produced from tomographic image information, during surgery in which a resection-use surgical instrument is used while an endoscopic image,wherein the surgery assistance program is used by a computer to execute a surgery assistance method comprising the steps of:

acquiring tomographic image information about a patient;
storing voxel information for the tomographic image information;
sampling voxel information in a direction perpendicular to the sight line on the basis of the voxel information;
sequentially sensing the three-dimensional positions of the endoscope and surgical instrument;
integrating the coordinates of the three-dimensional image and the coordinates of the endoscope and the surgical instrument;

storing the resection portion scheduled for surgery and virtually resected on the three-dimensional image, in a memory after associating it with the voxel information;

calculating the distance between the working end of the surgical instrument on the three-dimensional image and the voxel information indicating the resection portion stored in the memory; and

displaying the working end of the surgical instrument on the three-dimensional image by using the coordinates of the surgical instrument during surgery, and displaying the distance between the working end and the voxel information indicating the resection portion stored in the memory, along with the endoscopic image displayed during surgery.

10. A surgery assistance device configured to perform navigation while displaying a three-dimensional simulation image produced from tomographic image information, during surgery in which a resection-use surgical instrument is used while the user views an endoscopic image, the device comprising:

a simulator configured to store the resection portion scheduled for surgery and virtually resected on the three-dimensional image produced by sampling voxel information for the tomographic image information of the patient in a direction perpendicular to the sight line, after associating it with the voxel information; and

a navigator configured to calculate a distance between the working end of the surgical instrument on the three-dimensional image and the voxel information indicating the resection portion stored in the memory, display the working end of the surgical instrument on the three-dimensional image by using the coordinates of the surgical instrument during surgery, and display the distance between the working end and the voxel information indicating the resection portion, along with the endoscopic image displayed during surgery.

FIG. 1

FIG. 2

FIG. 3

Endoscope parameter storage section

(Ex.)

oblique angle

view angle

position

attitude

⋮

first endoscope parameter
storage section — 22a

second endoscope parameter
storage section — 22b

third endoscope parameter
storage section — 22c

— 22

# FIG. 4

EP 2 823 784 A1

Surgical instrument parameter
storage section

(Ex.: tubular retractor)

tubular shape

tube length

position

attitude

first surgical instrument
parameter storage section ———— 24a

second surgical instrument
parameter storage section ———— 24b

third surgical instrument
parameter storage section ———— 24c

———— 24

FIG. 5

EP 2 823 784 A1

32a

oblique endoscope
view angle

32

oblique angle

FIG. 6A

32a

32

FIG. 6B

26

## FIG. 7A

Start

Input tomographic image — S1

Extract voxel information — S2

Volume rendering — S3

Display rendered image — S4

Registration instruction? — S5
N / Y

Y → (A) — S6

N → Navigation instruction? — S7
Y → (B) — S8
N

## FIG. 7B

(A) — S6

Registration feature point instruction — S61

Acquire sensor position information — S62

Calculate conversion matrix — S63

Confirm registration accuracy — S64
NG / OK

OK → End A

## FIG. 7C

(B) — S8

Acquire endoscope/surgical instrument position and attitude — S81

Conversion by conversion matrix — S82

Acquire endoscope parameters — S83

Acquire surgical instrument parameters — S84

Acquire endoscope video — S85

Calculation of distance completed for all resection sites? — S86
N / Y

Superpose and display endoscope video and rendered image — S87

(C)

FIG. 8

EP 2 823 784 A1

FIG. 9

EP 2 823 784 A1

FIG. 10

EP 2 823 784 A1

FIG. 11

EP 2 823 784 A1

FIG. 12

FIG. 13A

FIG. 13B

display width H

mouse drag
distance Hd

2, 102

FIG. 14

FIG. 15

endoscope axis vector

endoscope axis vector

endoscope axis vector

sight line vector

sight line vector

sight line vector

FIG. 16A

FIG. 16B

FIG. 16C

FIG. 17

EP 2 823 784 A1

Oblique endoscope view (oblique perspective of 25°)

FIG. 18A

Direct-view endoscope view
(note: same viewpoint as diagram on left)

FIG. 18B

endoscope view

FIG. 19

EP 2 823 784 A1

FIG. 20A endoscopic image

FIG. 20B endoscope view

FIG. 20C superposed image

EP 2 823 784 A1

FIG. 21A endoscopic image

FIG. 21B VR image

FIG. 21C superposed image

FIG. 22

FIG. 23

FIG. 24A

FIG. 24B

33

surgical instrument (drill)

33b

(real space)

FIG. 25A

33a

layout of sampling points on the outside of the surgical instrument

(virtual space)

FIG. 25B

33a

Calculation and display of distance between
surgical instrument distal end and resection
site

Planned resection site

(virtual space)

FIG. 26

EP 2 823 784 A1

M

region expanded by distance $l_2$ from resection site

region expanded by distance $l_1$ from resection site

planned resection site

(virtual space)

FIG. 27

With control of threshold summing valid points

resection point

$\Delta D$

$\Delta D_{i-1}$ below specific value ($\Delta D_{i-1} < kTi$)
$\Rightarrow Ti$ not updated

resection executed

volume rendering image

resection range

depth change of resection point goes above threshold, resection cancelled

volume rendering image

(virtual space)

FIG. 28

EP 2 823 784 A1

$\Delta D_{i-1}$ is zero, Ti ≈ 0

Ti ≈ 0, resection ends up being cancelled for even a tiny depth change

resection point

$\Delta D$

volume rendering image

Without control of threshold summing valid points

mistaken resection cancellation

volume rendering image

(virtual space)

FIG. 29

132a

oblique endoscope
view angle

132

oblique angle

## FIG. 30A

132a

132

132b

## FIG. 30B

232c    232a

oblique endoscope
view angle

232

oblique angle

## FIG. 31A

232b
232a

232

232c

## FIG. 31B

## INTERNATIONAL SEARCH REPORT

| International application No. |
| --- |
| PCT/JP2013/002065 |

A. CLASSIFICATION OF SUBJECT MATTER
*A61B19/00*(2006.01)i, *A61B1/00*(2006.01)i, *A61B6/03*(2006.01)i, *G06T15/08*
(2011.01)i

According to International Patent Classification (IPC) or to both national classification and IPC

B. FIELDS SEARCHED

Minimum documentation searched (classification system followed by classification symbols)
A61B19/00, A61B1/00, A61B6/03, G06T15/08

Documentation searched other than minimum documentation to the extent that such documents are included in the fields searched
Jitsuyo Shinan Koho            1922-1996    Jitsuyo Shinan Toroku Koho    1996-2013
Kokai Jitsuyo Shinan Koho      1971-2013    Toroku Jitsuyo Shinan Koho    1994-2013

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y | WO 2011/102012 A1 (Olympus Medical Systems Corp.), 25 August 2011 (25.08.2011), entire text; all drawings & US 2011/0234780 A1     & EP 2377457 A1 | 1-10 |
| Y | JP 11-161813 A (Olympus Optical Co., Ltd.), 18 June 1999 (18.06.1999), entire text; all drawings & US 6283763 B1 | 1-10 |
| Y | WO 2011/118208 A1 (Panasonic Corp.), 29 September 2011 (29.09.2011), entire text; all drawings & US 2012/0032959 A1     & EP 2400463 A1 | 2-8 |

[X] Further documents are listed in the continuation of Box C.          [ ] See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 01 May, 2013 (01.05.13) | 14 May, 2013 (14.05.13) |

| Name and mailing address of the ISA/ Japanese Patent Office | Authorized officer |
| --- | --- |
| Facsimile No. | Telephone No. |

Form PCT/ISA/210 (second sheet) (July 2009)

**INTERNATIONAL SEARCH REPORT**

International application No.

PCT/JP2013/002065

C (Continuation).   DOCUMENTS CONSIDERED TO BE RELEVANT

| Category* | Citation of document, with indication, where appropriate, of the relevant passages | Relevant to claim No. |
|---|---|---|
| Y | Keiho IMANISHI, "Haptic Navigation Method for Improving Safety of Master-Slave Type Robotic Surgery", Transactions of the Virtual Reality Society of Japan, 30 September 2003 (30.09. 2003), vol.8, no.3 | 3-8 |
| Y | JP 2010-200894 A  (Tadashi UKIMURA), 16 September 2010 (16.09.2010), paragraphs [0038] to [0039]; fig. 4 (Family: none) | 5-8 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4152402 B